# EUROPEAN PATENT SPECIFICATION

(11) **EP 3 129 787 B1**
(45) Date of publication and mention of the grant of the patent: **20.03.2024**
(21) Application number: 15713925.4
(22) Date of filing: 08.04.2015
(51) Int. Cl.: G16H 50/50, G01N 33/68, G01N 33/576

(54) **PROGNOSTIC TESTS FOR HEPATIC DISORDERS**
PROGNOSTISCHE TESTS FÜR LEBERSTÖRUNGEN
TESTS PRONOSTIQUES POUR TROUBLES HÉPATIQUES

(30) Priority: 08.04.2014 EP 14163948
(43) Date of publication of application: 15.02.2017
(73) Proprietor: Centre Hospitalier Universitaire d'Angers, 49100 Angers (FR); Université d'Angers, 49000 Angers (FR)
(72) Inventor: CALÈS, Paul, 49240 Avrillé (FR); BOURSIER, Jérôme, 49000 Angers (FR); BERTRAIS, Sandrine, 49380 Thouarcé (FR)
(74) Representative: Icosa
(86) International application number: PCT/EP2015/057644
(87) International publication number: WO 2015/155259

(56) References cited:
- WO-A1-2010/106140
- JULIEN VERGNIOL ET AL: "Noninvasive Tests for Fibrosis and Liver Stiffness Predict 5-Year Outcomes of Patients With Chronic Hepatitis C", GASTROENTEROLOGY, ELSEVIER, PHILADELPHIA, PA, vol. 140, no. 7, 18 February 2011 (2011-02-18), pages 1970-1979.e3, XP028374902, ISSN: 0016-5085, DOI: 10.1053/J.GASTRO.2011.02.058 [retrieved on 2011-03-02]
- STERLING RICHARD K ET AL: "Development of a simple noninvasive index to predict significant fibrosis in patients with HIV/HCV coinfection", HEPATOLOGY (AASLD ABSTRACTS), JOHN WILEY & SONS, INC, USA, vol. 43, no. 6, 1 June 2006 (2006-06-01), pages 1317-1325, XP002563342, ISSN: 0270-9139, DOI: 10.1002/HEP.21178 [retrieved on 2006-05-25]
- J. BOURSIER ET AL: "Combination of blood tests for significant fibrosis and cirrhosis improves the assessment of liver-prognosis in chronic hepatitis C", ALIMENTARY PHARMACOLOGY & THERAPEUTICS, vol. 40, no. 2, 2 July 2014 (2014-07-02), pages 178-188, XP055139634, ISSN: 0269-2813, DOI: 10.1111/apt.12813

## Description

### FIELD OF INVENTION

The present invention relates to the prognosis of patients, especially patients suffering from hepatic disorders. More particularly, the present invention relates to a non-invasive prognostic method for assessing the risk of death or of liver-related event in a subject, wherein the subject is preferably affected with a hepatic disorder.

### BACKGROUND OF INVENTION

Epidemiological data highlight the significant burden of liver diseases worldwide, including in the United States and in Europe. The most common chronic liver diseases are caused by excessive alcohol consumption, or infections by the hepatitis C and B viruses, and an increasing proportion is related to obesity and metabolic syndrome (non-alcoholic fatty liver disease). Chronic liver diseases are important causes of morbidity and mortality.

In the prior art, interest was mainly focused on the diagnosis of these patients. The Applicant, for example, filed several patent applications on methods for assessing the presence and/or the severity of liver diseases. The Applicant also filed a patent application disclosing a non-invasive method for assessing liver fibrosis progression, said method relying on the use of the FibroMeter blood test (WO 2010/106140). Sterling et al (Hepatology 2006; 43(6): 1317-1325) developed a diagnosis model for predicting fibrosis. Said model, based on age, AST, platelet count and alanine aminotransferase (ALT), corresponds to the FIB-4 blood test. WO 2010/106140 and Sterling et al do not aim at predicting survival or assessing a risk of death and/or of liver-related event. These methods are diagnostic methods, i.e. elaborated with reference to liver biopsy and to a diagnostic target which is fibrosis, significant fibrosis or cirrhosis. In diagnostic methods, the relevancy of the biomarkers relative to the stage of fibrosis are assessed with comparison to the biopsies.

Diagnostic methods give efficient information on the severity of a fibrosis. However, there is a further need for help in making decisions for clinical monitoring of patients and for early identification of patients at risk of liver-related complications or death.

This further need could be fulfilled by prognostic methods, i.e. methods where the relevancy of the biomarkers is assessed on the basis of the life expectancy of the patients of the cohort of reference or on the basis of complications experienced by the patients of the cohort of reference.

In the prior art, different scores are correlated with the prognosis of cirrhosis, such as, for example the MELD score and the Child-Pugh score. The MELD score (wherein MELD stands for Model for End-Stage Liver Disease) consists in the mathematical combination of serum bilirubin and creatinine levels, International Normalized Ratio (INR) for prothrombin time and etiology of liver disease (Kamath et al, Hepatology 2001; 33:464-470). The Child-Pugh score is based on the combination of total bilirubin, serum albumin, International Normalized Ratio (INR) or prothrombin time, ascites and hepatic encephalopathy.

However, both the MELD and Child-Pugh scores are directed to the prognosis of cirrhosis only (Metavir F4 stage) and cannot help for prognostic of patients having lesser fibrosis degree like Metavir F0, F1, F2 or F3 fibrosis stages.

Other recent studies use diagnostic tests for the prediction of the survival in patients having alcoholic liver disease. For example, Naveau et al (Hepatology 2009, 49(1): 97-105) describe the use of the biomarkers of Fibrotest (a diagnostic score), in multivariate analysis, for manufacturing a prognostic score named Fibrotest prognostic.

Moreover, Vergniol et al (Gastroenterology 2011; 140: 1970-1979) studied the efficiency of liver stiffness, FibroTest, APRI or FIB-4 for predicting survival or liver-related death in hepatic disease patients. Vergniol et al concluded that liver stiffness and FibroTest may both be used for predicting survival or liver-related death, and that their mathematical combination presents a significant prognostic accuracy. However, there is no statistical difference between the accuracies of FibroTest, liver stiffness and their combination.

Furthermore, Vergniol et al does not describe or suggest a prognostic test for assessing the risk of liver-related event.

Applying diagnostic tests to prognosis may not outcome in the most accurate prognosis. This invention aims at proposing scores and methods intentionally designed for prognosis, on the basis of multivariate mathematical combination of blood biomarkers, clinical markers, tests, and/or physical data. The prognostic tests of the invention allow assessing the risk of death with an increased accuracy as compared to the prognostic tests of the prior art, and further allow assessing the risk of liver-related events.

### SUMMARY

The present invention relates to an in vitro non-invasive prognostic method for assessing the risk of death and/or of liver-related event in a subject, comprising:
a. obtaining at least three variables selected from age, sex, weight, alpha-2 macroglobulin (A2M), hyaluronic acid (HA), prothrombin index (PI), platelets (PLT), aspartate aminotransferase (AST), urea, gamma glutamyl transpeptidase (GGT), alanine aminotransferase (ALT), ferritin (Fer), and glucose (Glu);
b. obtaining at least one blood test result by multivariate combination, with a binary logistic regression, of the at least three variables obtained in step (a), said blood test being a FibroMeter,
c. obtaining at least one elastometry data from medical imaging or clinical measurement; and
d. mathematically combining in a multivariate time-related model:
   - said at least three variables obtained in step (a) and/or said at least one blood test result obtained in step (b); and
   - said at least one elastometry data obtained in step (c);
thereby obtaining a prognostic score.

In one embodiment, said at least one elastometry data is liver stiffness measurement (LSM). In one embodiment, said LSM is measured by Vibration Controlled Transient Elastography (VCTE).

In one embodiment, the method comprises mathematically combining in a multivariate time-related model in step (d) the following variables, or at least the following variables: a blood test result, said blood test being a FibroMeter, LSM, age, and sex.

In one embodiment, said multivariate time-related model is a Cox model, said multivariate Cox model being time-fixed or time-dependent. In one embodiment, said multivariate Cox model is time-dependent.

In one embodiment, death is all-cause death and/or liver-related death.

In one embodiment, said liver-related event is selected from ascites, encephalopathy, jaundice, occurrence of large esophageal varices, variceal bleeding, gastro-intestinal hemorrhage, hepato-renal syndrome, hepatocellular carcinoma, hepatic transplantation, esophageal varices, and portal hypertension.

In one embodiment, the subject is affected with a liver disease and/or the subject is a patient affected with a chronic disease. In one embodiment, the subject is affected with a liver disease selected from significant porto-septal fibrosis, severe porto-septal fibrosis, centrolobular fibrosis, cirrhosis, peri sinusoidal fibrosis, the fibrosis being of alcoholic or non-alcoholic origin. In one embodiment, the subject is a patient affected with a chronic disease selected from chronic viral hepatitis C, chronic viral hepatitis B, chronic viral hepatitis D, chronic viral hepatitis E, non-alcoholic fatty liver disease (NAFLD), alcoholic chronic liver disease, autoimmune hepatitis, primary biliary cirrhosis, hemochromatosis and Wilson disease.

In one embodiment, the method of the invention is computer implemented.

Another object of the invention is a microprocessor comprising a computer algorithm for carrying out the in vitro method of the invention.

### DEFINITIONS

In the present invention, the following terms have the following meanings:
- "Prognosis" refers to the statistical prediction of the occurrence of death or of the occurrence of a liver-related event (or of the first liver-related event) over a specific time period for a subject, such as, for example, within a period of at least 3 months, preferably 3 months, 6 months, 9 months, or 1, 2, 3, 4, 5, 6, 7, 8, 9, or 10 years as of date of assessment. The term "prognosis" thus relates to the assessment of a specific risk of death or of liver-related event for a subject.
- "Liver-related event" refers to a liver-related event or complication requiring specific therapy or care or to the progression of the liver disease or disorder in a patient, including, without limitation, jaundice, ascites, encephalopathy, variceal bleeding, hepatocellular carcinoma, severe infection (like septic shock), hepato-renal syndrome, hepato-pulmonary syndrome.
- "Risk" in the context of the present invention, relates to the probability that death or a liver-related event will occur over a specific time period, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(1-p) wherein p is the probability of event and (1- p) is the probability of no event). In one embodiment, the method of the invention may further comprise a step of comparing the prognostic score obtained from the subject with a reference score.
- "Score" refers to any digit value obtained by the mathematical combination (univariate or multivariate) of at least one biomarker and/or at least one clinical data and/or at least one physical data and/or at least one binary marker and/or at least one blood test result. In one embodiment, a score is an unbound digit value. In another embodiment, a score is a bound digit value, obtained by a mathematical function. Preferably, a score ranges from 0 to 1. In one embodiment, the at least one biomarker and/or at least one clinical data and/or at least one physical data and/or at least one binary marker and/or at least one score, mathematically combined in a score are independent, i.e. give each information that is different and not linked to the information given by the others.
- "About" preceding a figure means plus or less 10% of the value of said figure.
- "Biomarkers" refers to a variable that may be measured in a sample from the subject, wherein the sample may be a bodily fluid sample, such as, for example, a blood, serum or urine sample, preferably a blood or serum sample.
- "Clinical data" refers to a data recovered from external observation of the subject, without the use of laboratory tests and the like.
- "Binary marker" refers to a marker having the value 0 or 1 (or yes or no).
- "Physical data" refers to a variable obtained by a physical method.
- "Blood test" corresponds to a test comprising non-invasively measuring at least one data, and, when at least two data are measured, mathematically combining said at least two data within a score. In the present invention, said data may be a biomarker, a clinical data, a physical data, a binary marker or any combination thereof (such as, for example, any mathematical combination within a score).

### DETAILED DESCRIPTION

The present invention relates to a non-invasive *in vitro* prognostic method for prognosing death (preferably liver-related death) or liver-related events, especially complications, in a subject. Accordingly, the method of the invention may also be defined as an *in vitro* method for assessing the risk of death or of liver-related events, especially liver-related complications, in a subject. The present invention also relates to a non-invasive *in vitro* prognostic method for determining an increased risk of mortality or of liver-related event in a subject. The predictive power of the prognosis method of the invention is assessed by the Harrell's C index which reflects the discriminative ability.

In one embodiment, the prognosis method of the invention has a Harrell's C index of at least about 0.70, preferably of at least about 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99 or more.

The present invention thus relates to an *in vitro* prognostic method, said method being non-invasive, for assessing the risk of death and/or of liver-related event, especially complication, in a subject, comprising:
a. obtaining at least three variables selected from age, sex, weight, alpha-2 macroglobulin (A2M), hyaluronic acid (HA), prothrombin index (PI), platelets (PLT), aspartate aminotransferase (AST), urea, gamma glutamyl transpeptidase (GGT), alanine aminotransferase (ALT), ferritin (Fer), and glucose (Glu);
b. obtaining at least one blood test result by multivariate combination, with a binary logistic regression, of the at least three variables obtained in step (a), said blood test being a FibroMeter:
c. obtaining at least one physical data from medical imaging or clinical measurement, said physical data being an elastometry data, preferably from Vibration Controlled Transient Elastography (VCTE), and
d. mathematically combining in a multivariate time-related model:
   - said at least three variables obtained in step (a) and/or said at least one blood test result obtained in step b); and
   - said at least one physical data, obtained in step (c);
thereby obtaining a prognostic score.

According to the invention, said blood test obtained in step (b) is not an APRI, FIB-4 or Actitest.

In one embodiment, at least 3, 4, 5, 6, 7, 8 or more variables are obtained in step (a).

In one embodiment, the method of the invention further comprises a step of assessing the risk of death and/or of liver-related event, especially complication, in the subject according to the value of the prognostic score obtained in step (d).

In one embodiment, the method of the invention comprises mathematically combining in a multivariate time-related model in step (d) said at least three variables obtained in step (a) and said at least one physical data obtained in step (c).

In another embodiment, the method of the invention comprises mathematically combining in a multivariate time-related model in step (d) said at least one blood test obtained in step (b) and said at least one physical data obtained in step (c).

In one embodiment, the terms "death" and "mortality" both refer to overall death or mortality (which may also be referred as all-cause death or mortality) and/or to liver-related death or mortality. Examples of causes of liver-related deaths include, but are not limited to, deaths consecutive to a portal hypertension related hemorrhage, deaths consecutive to an esophageal or gastric varice related hemorrhage, a hepatocellular carcinoma, ascites, encephalopathy, liver failure with sepsis, acute on chronic liver failure, hepato-renal syndrome, hepato-pulmonary syndrome or other liver decompensation.

In one embodiment, the term "liver-related event, especially complications" refers to a liver-related event or complication requiring specific therapy or care, such as, for example, ascites, encephalopathy, jaundice (which may be defined as serum bilirubin > 50 µmol/l), occurrence of large esophageal varices (preferably having a diameter ≥ 5 mm, and/or preferably a diameter ≥ 25 % of esophageal circumference), variceal bleeding, gastro-intestinal hemorrhage (such as, for example, due to portal hypertension), hepatorenal syndrome, hepato-pulmonary syndrome, hepatocellular carcinoma, hepatic transplantation, esophageal varices, portal hypertension superior or equal to a predetermined threshold (such as, for example, hepatic vein pressure gradient superior or equal to 10 mm Hg or superior or equal to 12 mm Hg), severe infection (such as, for example, septic shock).

In one embodiment, the term "liver-related event, especially complication" refers to the progression of the liver disease or disorder in a patient, such as, for example, the appearance of cirrhosis in a fibrotic non-cirrhotic patient, or the fact, for a patient, to cross a predetermined threshold (such as, for example, FibroMeter result superior or equal to 0.982, or Fibroscan result superior or equal to 14 kPa).

According to the invention, death (including all-cause death and liver-related death) does not refer to a liver-related event, especially complication.

In one embodiment, the method of the invention is for prognosing the first liver-related event, especially complication, in a patient.

In one embodiment of the invention, the prognostic method of the invention is for assessing the risk of death or of a liver-related event within a period of at least 3 months, preferably 3 months, 6 months, 9 months, 1, 2, 3, 4, 5, 6, 7, 8, 9, or 10 years as of date of assessment.

In one embodiment, the subject is a mammal, preferably a human. In one embodiment, the subject is a male or a female. In one embodiment, the subject is an adult or a child.

In one embodiment, the subject is affected, preferably is diagnosed with a liver disease or disorder, and the method of the invention may thus be defined as a method of prognosing liver disease or disorder.

Preferably, the subject is a patient, i.e. the subject is awaiting the receipt of, or is receiving medical care or is/will be the object of a medical procedure.

In one embodiment, the subject is affected with a liver disease or disorder, preferably selected from the list comprising significant porto-septal fibrosis, severe porto-septal fibrosis, centrolobular fibrosis, cirrhosis, peri sinusoidal fibrosis, the fibrosis being from alcoholic or non-alcoholic origin.

In one embodiment, the subject is a patient affected with a chronic disease, preferably said chronic disease is selected from the group comprising chronic viral hepatitis C, chronic viral hepatitis B, chronic viral hepatitis D, chronic viral hepatitis E, non-alcoholic fatty liver disease (NAFLD), alcoholic chronic liver disease, autoimmune hepatitis, primary biliary cirrhosis, hemochromatosis and Wilson disease.

Examples of biomarkers include glycemia, total cholesterol, HDL cholesterol (HDL), LDL cholesterol (LDL), AST (aspartate aminotransferase), ALT (alanine aminotransferase), AST/ALT (ratio), AST.ALT (product), ferritin, platelets (PLT), AST/PLT (ratio), prothrombin time (PT) or prothrombin index (PI), hyaluronic acid (HA or hyaluronate), hemoglobin, triglycerides, alpha-2 macroglobulin (A2M), gamma-glutamyl transpeptidase (GGT), urea, bilirubin, apolipoprotein A1 (ApoA1), type III procollagen N-terminal propeptide (P3NP or P3P), gamma-globulins (GBL), sodium (Na), albumin (ALB), ferritin (Fer), glucose (Glu), alkaline phosphatases (ALP), YKL-40 (human cartilage glycoprotein 39), tissue inhibitor of matrix metalloproteinase 1 (TIMP-1), TGF, cytokeratin 18 and matrix metalloproteinase 2 (MMP-2) to 9 (MMP-9), haptoglobin, ratios and mathematical combinations thereof.

Examples of clinical data include, but are not limited to, weight, body mass index, age, sex, hip perimeter, abdominal perimeter or height and mathematical combinations thereof, such as, for example, the ratio thereof, such as for example hip perimeter/abdominal perimeter.

Examples of binary non-invasive test markers include diabetes, SVR (wherein SVR stands for sustained virologic response, and is defined as aviremia 6 weeks, preferably 12 weeks, more preferably 24 weeks after completion of antiviral therapy for chronic hepatitis C virus (HCV) infection), etiology, and NAFLD. Regarding the binary marker "etiology", the skilled artisan knows that said variable is a single or multiple binary marker, and that for liver disorders, etiology may be NAFLD, alcohol, virus or other. Thus, the binary marker might be expressed as NAFLD vs others (single binary marker) or as NAFLD vs reference etiology plus virus vs reference etiology and so on (multiple binary marker).

Examples of blood tests include ELF, FibroSpect^{™}, APRI, FIB-4, Hepascore, Fibrotest^{™}, FibroMeter^{™}, CirrhoMeter^{™}, CombiMeter, Elasto-FibroMeter, InflaMeter^{™}, Elasto-Fibrotest. As these blood tests are diagnostic tests, they are based on multivariate mathematical combination, such as, for example, binary logistic regression.

ELF is a blood test based on hyaluronic acid, P3P, TIMP-1 and age.

FibroSpect^{™} is a blood test based on hyaluronic acid, TIMP-1 and A2M.

APRI is a blood test based on platelet and AST.

FIB-4 is a blood test based on platelet, AST, ALT and age.

HEPASCORE is a blood test based on hyaluronic acid, bilirubin, alpha2-macroglobulin, GGT, age and sex.

FIBROTEST^{™} is a blood test based on alpha2-macroglobulin, haptoglobin, apolipoprotein A1, total bilirubin, GGT, age and sex.

ELASTO-FIBROTEST is a test based on the mathematical combination of variables of FIBROTEST or of the result of a FIBROTEST, with LSM measurement, measured for example by Fibroscan^{™}.

FIBROMETER^{™} and CIRRHOMETER^{™} together form a family of blood tests, the content of which depends on the cause of chronic liver disease and the diagnostic target (such as, for example, fibrosis, significant fibrosis (moderate bridging fibrosis), severe fibrosis (extensive bridging fibrosis) or cirrhosis). This blood test family is called FM family and is detailed in the table below.

| **Cause** | **Variables** | | | | | | | | | | | | |
|---|---|---|---|---|---|---|---|---|---|---|---|---|---|
| | Age | Sex | Weigth | A2M | HA | PI | PLT | AST | Urea | GGT | ALT | Fer | Glu |
| **Virus** | | | | | | | | | | | | | |
| FM V 1G | X | | | x | X | X | X | X | X | | | | |
| FM V 2G | X | X | | X | X | X | X | X | X | | | | |
| CM V 2G | X | X | | X | X | X | X | X | X | | | | |
| FM V 3G^{a} | X | X | | X | | X | X | X | X | X | | | |
| CM V 3G^{a} | X | X | | X | | X | X | X | X | X | | | |

| **Alcohol** | | | | | | | | | | | | | |
|---|---|---|---|---|---|---|---|---|---|---|---|---|---|
| FM A 1G | X | | | X | X | X | | | | | | | |
| FM A 2G | | | | x | X | X | | | | | | | |

| **NAFLD** (steatosis) | | | | | | | | | | | | | |
|---|---|---|---|---|---|---|---|---|---|---|---|---|---|
| FMS | X | | X | | | | X | X | | | X | X | X |

| | | | | | | | | | | | | | |
|---|---|---|---|---|---|---|---|---|---|---|---|---|---|
| FM: FibroMeter, CM: CirrhoMeter, A2M: alpha-2 macroglobulin, HA: hyaluronic acid, PI: prothrombin index, PLT: platelets, Fer: ferritin, Glu: glucose ^{a} HA may be replaced by GGT | | | | | | | | | | | | | |

COMBIMETER or Elasto-FibroMeter is a family of tests based on the mathematical combination of variables of the FM family (as detailed in the Table hereinabove) or of the result of a test of the FM family with FIBROSCAN^{™} result.

For example, CombiMeter or Elasto-FibroMeter results in a score based on the mathematical combination of physical data from liver or spleen elastometry such as dispersion index from Fibroscan^{™} such as IQR or IQR/median or median, preferably of Fibroscan^{™} median with at least 3, preferably at least 4, 5, 6, 7 or more and more preferably of 7 or 8 or 9 biomarkers and/or clinical data selected from the list comprising glycemia, total cholesterol, HDL cholesterol (HDL), LDL cholesterol (LDL), AST (aspartate aminotransferase), ALT (alanine aminotransferase), AST/ALT, AST.ALT, ferritin, platelets (PLT), AST/PLT, prothrombin time (PT) or prothrombin index (PI), hyaluronic acid (HA or hyaluronate), hemoglobin, triglycerides, alpha-2 macroglobulin (A2M), gamma-glutamyl transpeptidase (GGT), urea, bilirubin, apolipoprotein A1 (ApoA1), type III procollagen N-terminal propeptide (P3NP), gamma-globulins (GBL), sodium (Na), albumin (ALB), ferritin (Fer), glucose (Glu), alkaline phosphatases (ALP), YKL-40 (human cartilage glycoprotein 39), tissue inhibitor of matrix metalloproteinase 1 (TIMP-1), TGF, cytokeratin 18 and matrix metalloproteinase 2 (MMP-2) to 9 (MMP-9), haptoglobin, diabetes, weight, body mass index, age, sex, hip perimeter, abdominal perimeter or height and ratios and mathematical combinations thereof.

INFLAMETER^{™} is a companion test reflecting necro-inflammatory activity including ALT, A2M, PI, and platelets.

The method of the invention does not comprise obtaining a Fibrostest^{™} in step (a).

The method of the invention does not comprise obtaining an Actitest^{™} in step (a) and/or in step (b). ACTITEST^{™} is a blood test based on alpha2-macroglobulin, haptoglobin, apolipoprotein A1, total bilirubin, GGT, ALT, age and sex.

The method of the invention does not comprise obtaining an APRI in step (a) and/or in step (b).

The method of the invention does not comprise obtaining a FIB-4 in step (a) and/or in step (b).

The method of the invention does not comprise or consists in mathematically combining in a multivariate domain a Fibrotest^{™} result and a physical data, such as, for example, liver stiffness measurement (LSM) measured by VCTE (such as, for example, Fibroscan).

In one embodiment, the method of the invention does not comprise obtaining in step (a) and mathematically combining in a multivariate time-related model with a physical data in step (d) the variables of APRI, i.e. platelet and AST.

In one embodiment, the method of the invention comprises obtaining in step (a) and mathematically combining in a multivariate time-related model with a physical data in step (d) the variables of FIB-4, i.e. platelet, ASAT, ALT and age. In one embodiment, the method of the invention does not comprise or consist in obtaining in step (a) and mathematically combining in a multivariate time-related model with a physical data in step (d) the variables of FIB-4, i.e. platelet, ASAT, ALT and age.

In one embodiment, the method of the invention comprises obtaining in step (a) and mathematically combining in a multivariate time-related model with a physical data in step (d) the variables of HEPASCORE, i.e. hyaluronic acid, bilirubin, alpha2-macroglobulin, GGT, age and sex.

In one embodiment, the method of the invention comprises obtaining in step (a) and mathematically combining in a multivariate time-related model with a physical data in step (d) the variables of FIBROTEST^{™}, i.e. alpha2-macroglobulin, haptoglobin, apolipoprotein A1, total bilirubin, GGT, age and sex. In one embodiment, the method of the invention does not comprise or consist in obtaining in step (a), and mathematically combining in a multivariate time-related model with a physical data in step (d) the variables of FIBROTEST^{™}, i.e. alpha2-macroglobulin, haptoglobin, apolipoprotein A1, total bilirubin, GGT, age and sex.

In one embodiment, the method of the invention comprises obtaining in step (a) and mathematically combining in a multivariate time-related model with a physical data in step (d) the variables of ACTITEST^{™}, i.e. alpha2-macroglobulin, haptoglobin, apolipoprotein A1, total bilirubin, GGT, ALT, age and sex. In one embodiment, the method of the invention does not comprise or consist in obtaining in step (a), and mathematically combining in a multivariate time-related model with a physical data in step (d) the variables of ACTITEST^{™}, i.e. alpha2-macroglobulin, haptoglobin, apolipoprotein A1, total bilirubin, GGT, ALT, age and sex.

In one embodiment, the method of the invention comprises obtaining in step (a) and mathematically combining in a multivariate time-related model with a physical data in step (d) the variables of FIBROMETER^{™} and/or CIRRHOMETER^{™} as listed in the Table hereinabove.

In one embodiment, the method of the invention comprises obtaining in step (a) and mathematically combining in a multivariate time-related model with a physical data in step (d) the variables of COMBIMETER or Elasto-FibroMeter as presented hereinabove.

In one embodiment, the method of the invention comprises obtaining in step (a) and mathematically combining in a multivariate time-related model with a physical data in step (d) the variables of Elasto-Fibrotest as presented hereinabove.

In one embodiment, the method of the invention comprises obtaining in step (a) and mathematically combining in a multivariate time-related model with a physical data in step (d) the variables of INFLAMETER^{™}, i.e. ALT, A2M, PI, and platelets.

Examples of physical methods include medical imaging data and clinical measurements, such as, for example, measurement of spleen, especially spleen length. According to an embodiment, the physical method is selected from the group comprising ultrasonography, especially Doppler-ultrasonography and elastometry ultrasonography and velocimetry ultrasonography (preferred tests using said data are vibration controlled transient elastography (VCTE, also known as Fibroscan^{™}), ARFI, VTE, supersonic elastometry (supersonic imaging), MRI (Magnetic Resonance Imaging), and MNR (Magnetic Nuclear Resonance) as used in spectroscopy, especially MNR elastometry or velocimetry.

In one embodiment, the physical method is VCTE, ARFI, VTE, supersonic elastometry or MRI stiffness.

According to the invention, the data is an elastometry data, preferably Liver Stiffness Evaluation (LSE) data or spleen stiffness evaluation, which may be for example obtained by VCTE. According to a preferred embodiment of the invention, the physical data is liver stiffness measurement (LSM), preferably measured by VCTE.

In a particular embodiment, the physical data is Liver stiffness measurement (LSM) by VCTE (also known as Fibroscan^{™}, Paris, France), preferably performed with the M probe. Preferably, examination conditions are those recommended by the manufacturer, with the objective of obtaining at least 3 and preferably 10 valid measurements. Results may be expressed as the median (kilopascals) of all valid measurements, or as IQR or as the ratio (IQR/median).

In one embodiment, the method of the invention comprises mathematically combining in a multivariate time-related model in step (d) prothrombin index, bilirubin, urea, hyaluronic acid, LSM, age and sex.

In one embodiment, the method of the invention comprises mathematically combining in a multivariate time-related model in step (d) age, sex, etiology, LSM, AST and urea.

In one embodiment, the method of the invention comprises mathematically combining in a multivariate time-related model in step (d) age, sex, NAFLD, LSM, AST and urea.

In one embodiment, the method of the invention comprises mathematically combining in a multivariate time-related model in step (d) age, sex, LSM, AST and urea.

In one embodiment, the method of the invention comprises mathematically combining in a multivariate time-related model in step (d) age, sex, NAFLD, prothrombin time, bilirubin, urea and LSM.

In one embodiment, the method of the invention comprises mathematically combining in a multivariate time-related model in step (d) age, sex, NAFLD, prothrombin time, AST, urea and LSM.

In one embodiment, the method of the invention comprises mathematically combining in a multivariate time-related model in step (d) age, sex, prothrombin time, bilirubin, urea, hyaluronic acid and LSM.

In one embodiment, the method of the invention comprises mathematically combining in a multivariate time-related model in step (d) age, sex, prothrombin time, bilirubin, urea and LSM.

In one embodiment, the method of the invention comprises mathematically combining in a multivariate time-related model in step (d) age, sex, prothrombin time, AST, urea and LSM.

In one embodiment, the method of the invention comprises mathematically combining in a multivariate time-related model in step (d) age, sex, etiology, AST, urea and LSM.

In one embodiment, said multivariate time-related model is a Cox proportional hazard regression model.

In one embodiment, the prognostic score of the invention is based on time-fixed Cox model, i.e. the method of the invention comprises only one measurement of variables selected from biomarkers, clinical data, binary markers and blood tests and/or physical data, which are generally recorded at baseline time.

In another embodiment, the prognostic score of the invention is based on time-dependent Cox model, i.e. the method of the invention comprises at least two successive measurements of variables selected from biomarkers, clinical data, binary markers and blood tests and/or physical data recorded at various times including baseline time.

In one embodiment, the prognosis method of the invention further comprises a step of comparing the prognostic score obtained for the subject with a reference score.

According to the invention, the prognostic score is indicative of the risk of death or of liver-related event in the subject.

In one embodiment, the prognostic method of the invention further comprises comparing the prognostic score obtained for the subject with a reference prognostic score. In one embodiment, a difference or an absence of difference between the prognostic score obtained for the subject and the reference score is indicative of an increased risk of death or of liver-related events.

In one embodiment, the reference score may be an index score or may be derived from one or more risk prediction algorithms or computed indices for the prognosis of death or liver-related events. A reference score can be relative to a number or value derived from population studies, including without limitation, such subjects having similar fibrosis level, subjects of the same or similar age range, subjects in the same or similar ethnic group.

In one embodiment of the present invention, the reference score is derived from one or more subjects (i.e. the reference population) who are substantially healthy, i.e. who are not affected with a liver disease or disorder. In another embodiment of the present invention, the reference score is derived from one or more subjects (i.e. the reference population) who are affected with a liver disease or disorder.

In one embodiment of the invention, a prognostic score obtained for the subject which is different from the reference score is indicative of a risk of death or of liver-related event. Preferably, in this embodiment, the reference score is derived from substantially healthy subjects.

In another embodiment of the invention, a prognostic score obtained for the subject which is not different from the reference score is indicative of a risk of death or of liver-related event. Preferably, in this embodiment, the reference score is derived from liver disease patients, more preferably from liver disease patients with a poor prognosis.

In one embodiment, a first value is considered different from a second value if the first one is superior or inferior by about 10%, 20%, 30%, 40%, 50%, 60%, 70%, 80%, 90%, 100% or more compared to the second one.

In one embodiment, the prognostic scores measured in the reference population used for obtaining the reference score are classified in percentiles, wherein the prognostic scores obtained by all subjects of the reference population are ranged according to their numerical value in ascending order. In one embodiment of the invention, the percentiles are percentiles of subjects, i.e. each percentile comprises the same number of subjects. Therefore, the first percentile corresponds to subjects with the lowest prognostic scores, while the last percentile corresponds to subjects with the highest prognostic scores.

In one embodiment, the reference score corresponds to the highest prognostic score of the first percentile of the reference population.

In another embodiment, the reference score corresponds to the highest prognostic score of the second, third... or penultimate percentile of the reference population.

In one embodiment, when three percentiles are drawn, each percentile is named a tertile. According to this embodiment, the reference score corresponds to the highest prognostic score of the first or of the second tertile.

In another embodiment, when four percentiles are drawn, each percentile is named a quartile. According to this embodiment, the reference value corresponds to the highest prognostic score of the first, second or third quartile.

In another embodiment, when five percentiles are drawn, each percentile is named a quintile. According to this embodiment, the reference value corresponds to the highest prognostic score of the first, second, third or fourth quintile.

In one embodiment of the invention, the prognostic method of the invention thus leads to the classification of the subject in a class of a classification, wherein each class of the classification corresponds to a specific risk of death or liver-related event. Such classifications, wherein a specific prognostic score corresponds to a specific assessed risk, are also objects of the present invention.

For example, the prognostic method of the invention leads to the classification of the subject in a classification comprising 5 classes, each corresponding to a quintile: the first class (or quintile) corresponds to a risk of death or of liver-related event of 0% in 5 years, the second class (or quintile) corresponds to a risk of death or of liver-related event superior to 0% and inferior or equal to 25%, the third class (or quintile) corresponds to a risk of death or of liver-related event superior to 25% and inferior or equal to 50%, the fourth class (or quintile) corresponds to a risk of death or of liver-related event superior to 50% and inferior or equal to 75%, and the fifth and last class (or quintile) corresponds to a risk of death or of liver-related event superior to 75%.

In one embodiment of the invention, the prognostic method of the invention thus leads to the classification of the subject in a class of a combined classification (which may also be referred to as a score risk chart), wherein said combined classification is in the form of a table wherein each cell is associated with a specific assessed risk of death or liver-related event.

In one embodiment, the score risk chart is a matrix table with 2 dimensions, wherein each dimension corresponds to a blood test and/or a physical method, for which a classification was previously constructed with the use of a reference to a reference population. For example, carrying out a Fibroscan may result in the classification of a subject in one of 3 fibrosis stages (F0-F1, F2-F3 or F4). Therefore, the first dimension of the score risk chart (lines) corresponds to stages for the first blood test or physical method, while the second dimension (columns) corresponds to stages for the second blood test or physical method.

In one embodiment, the score risk chart comprises additional dimensions, corresponding for example to clinical data, such as, for example, age and sex.

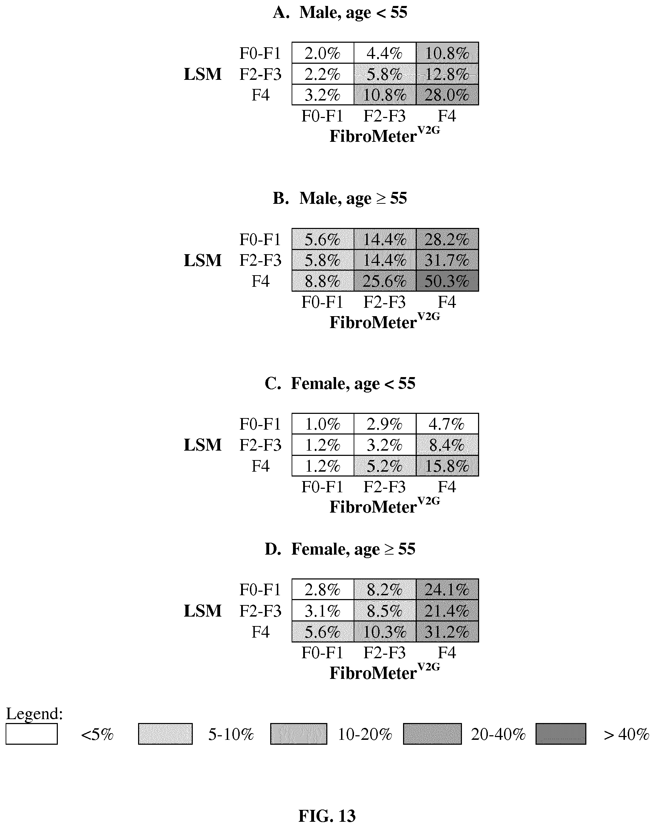

An example of 4-dimensions score risk chart is shown in **Figure 13****,** resulting from the combination of Fibroscan (LSM), FibroMeter, age and sex, wherein each case of the risk chart corresponds to a percentage risk of all-cause death before 3 years.

In one embodiment of the invention, the method of the invention is computer implemented.

The present invention thus also relates to a microprocessor comprising a computer algorithm carrying out the prognostic method of the invention.

The skilled artisan would easily deduce that the prognostic score of the invention being indicative of a particular risk of death or of liver-related event, it may be used by the physician willing to provide the best medical care to his/her patient. For example, a patient presenting a high risk of death or liver-related event will require more frequent follow-up than a patient with low risk.

Moreover, the prognostic score of the invention may be used for prioritizing patients for hepatic transplantation.

### BRIEF DESCRIPTION OF THE DRAWINGS

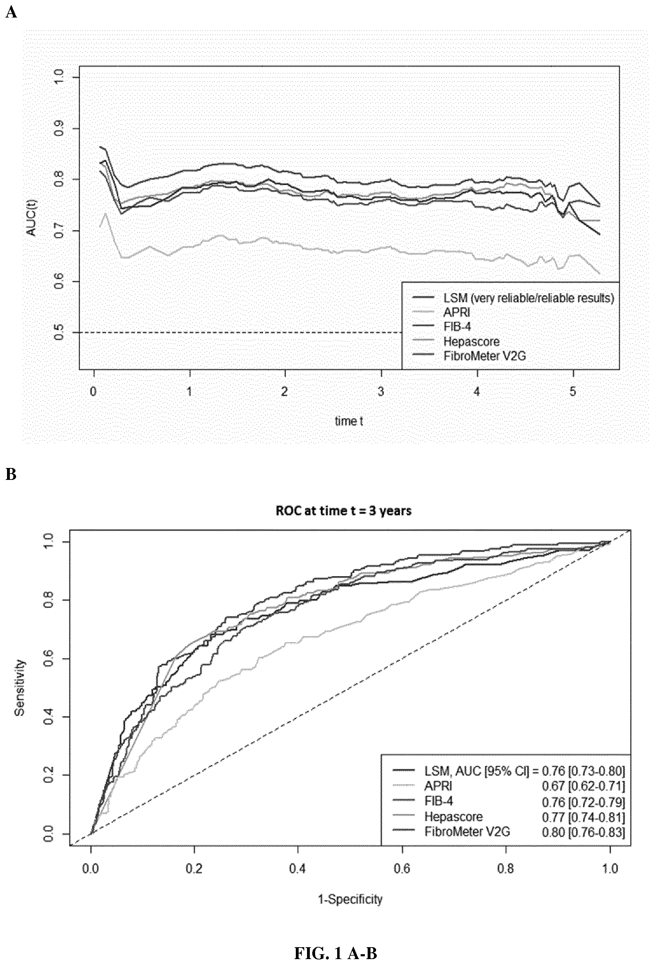

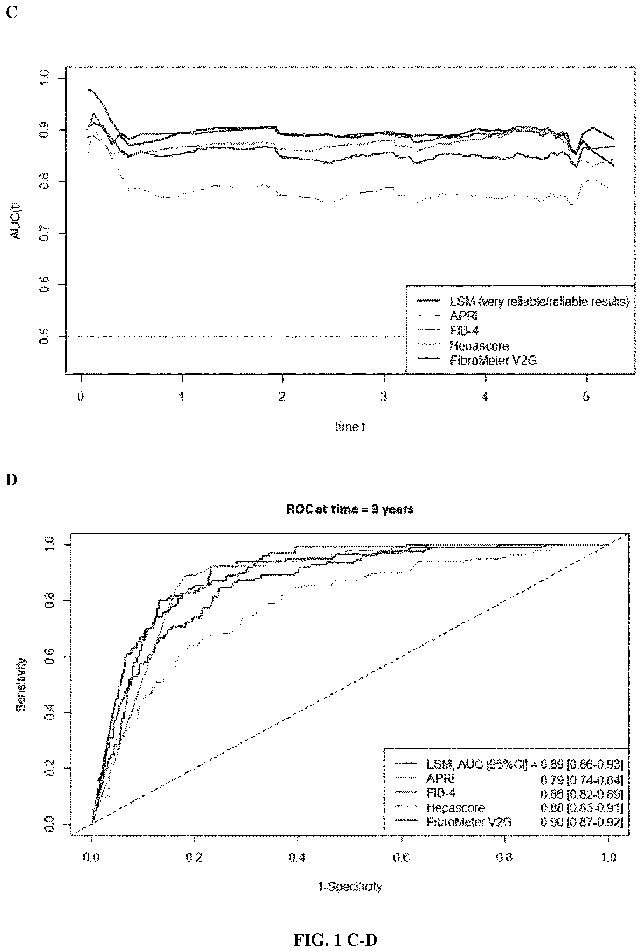

**Figure 1** is a combination of graphs, showing time-dependent AUROC(t) (A, C) and sensitivity (B, D) of each non-invasive liver fibrosis test for predicting all-cause mortality (A, B) and liver-related mortality (C, D) in the 1565 patients having available data for all non-invasive liver fibrosis tests within a 3-months interval at inclusion.

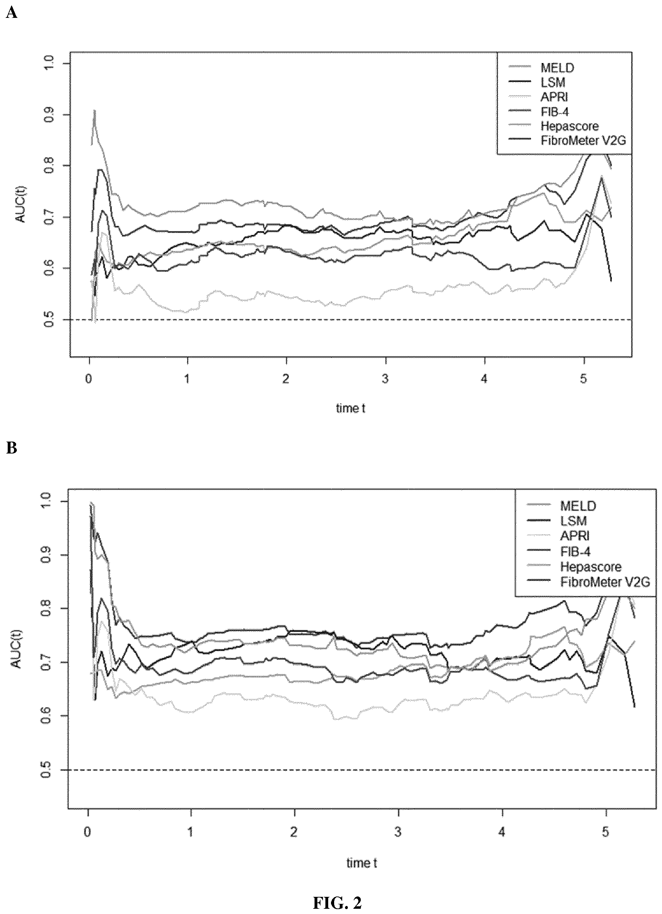

**Figure 2** is a combination of graphs showing time-dependent AUROC(t) of each non-invasive liver fibrosis test and MELD score for predicting overall mortality (A) and liver-related mortality (B) in 444 patients being cirrhotic at baseline, as classified by LSM ≥ 14 kPa.

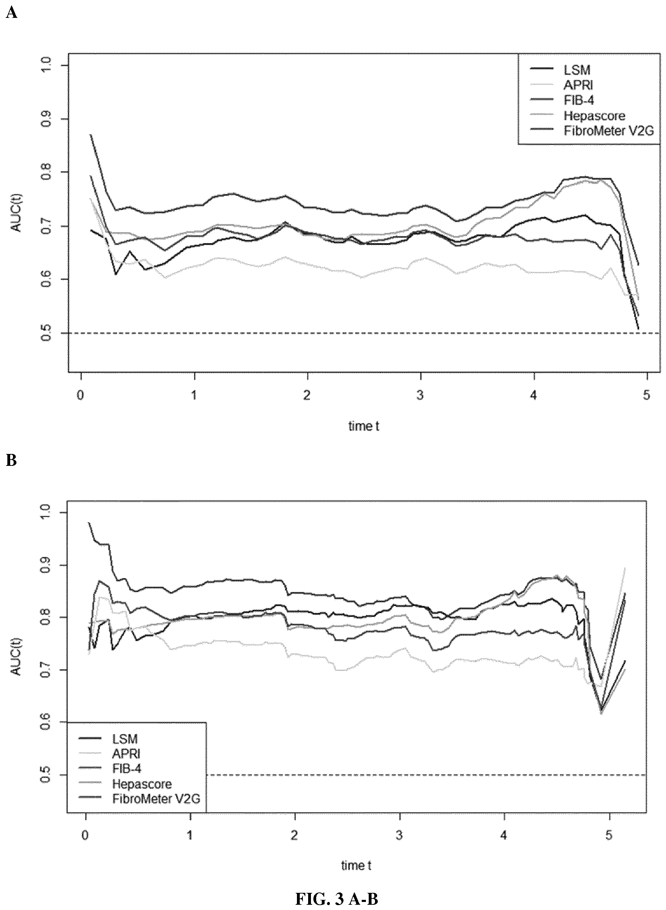

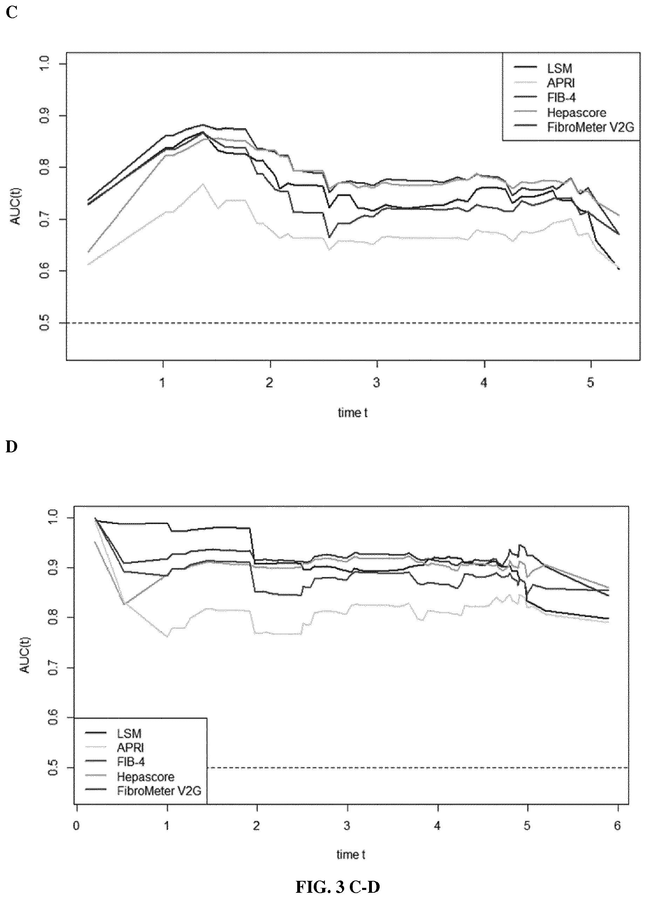

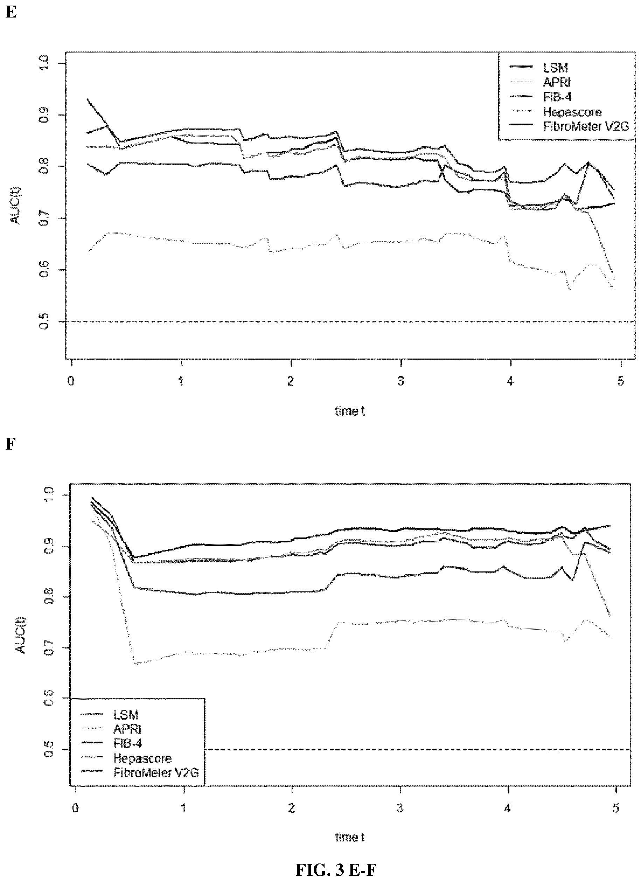

**Figure 3** is a combination of graphs showing time-dependent AUROC(t) of each non-invasive liver fibrosis test for predicting overall mortality (A (643 patients, 181 deaths), C (478 patients, 35 deaths) and E (313 patients, 39 deaths)) and liver-related mortality (B (642 patients, 85 deaths), D (474 patients, 19 deaths) and F (311 patients, 10 deaths)) according to the etiology of chronic liver disease: (A-B) alcoholic liver disease, (C-D) chronic viral hepatitis and (E-F) non-alcoholic fatty liver disease.

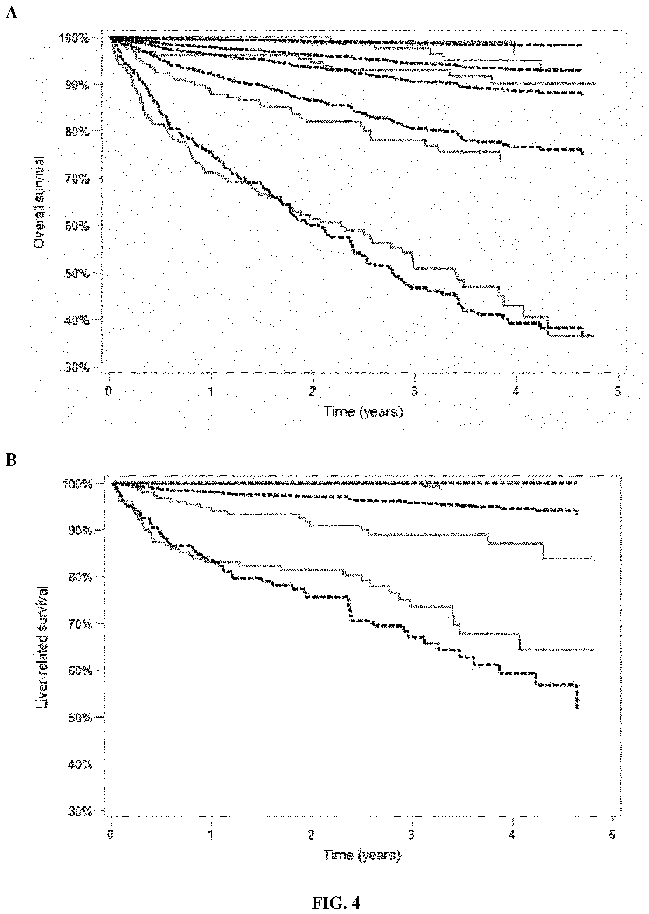

**Figure 4** is a combination of graphs showing the evaluation of calibrative ability of the time-fixed multivariate models, including age, sex, baseline results of LSM and FibroMeter^{v2G}, in the validation group (n=782). Observed (continuous line) and estimated (dotted line) survival rates of the patients divided into 5 groups according to their risk score for all-cause death (A) and for liver-related death (B). For liver-related mortality, the first three quintiles were combined due to the small number of events. The first upper curve corresponds to the first quintile and so on.

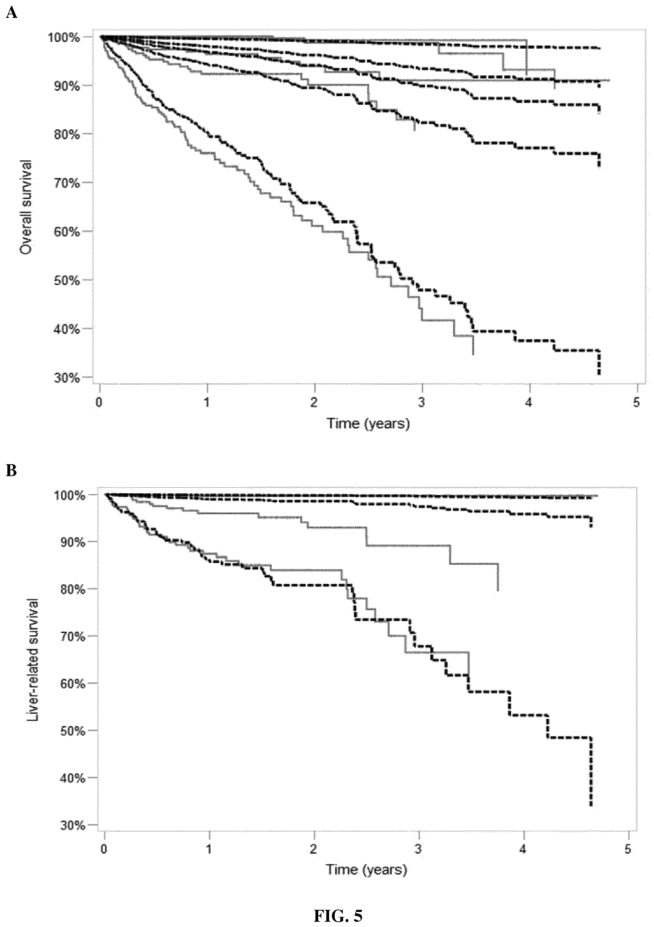

**Figure 5** is a combination of graphs showing the evaluation of calibrative ability of the time-dependent multivariate models, including age, sex, time-varying results of LSM and FibroMeter^{v2G}, in the validation group (782 patients, 1342 survival data points). Observed (continuous line) and estimated (dotted line) survival rates of the patients divided into 5 groups according to their risk score for all-cause death (A) and for liver-related death (B). For liver-related mortality, the first three quintiles were combined due to the small number of events.

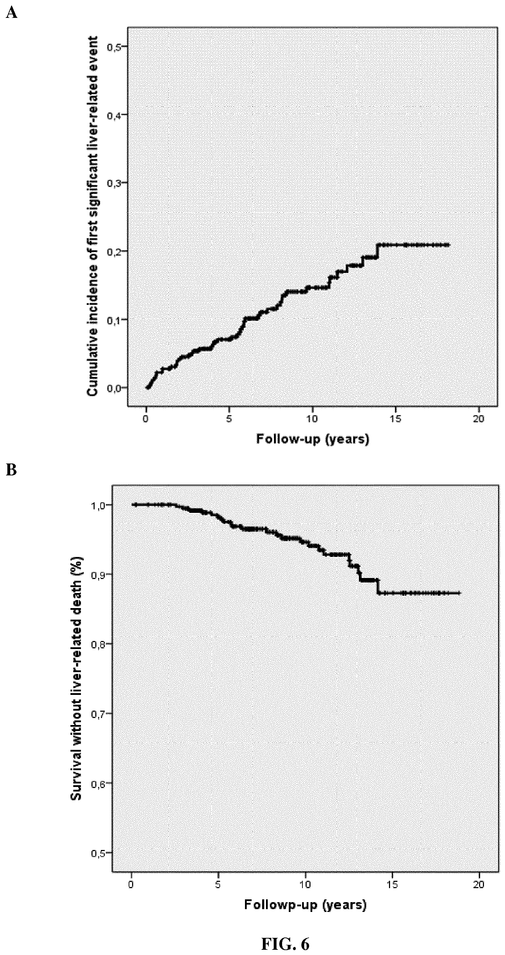

**Figure 6** is a combination of graphs showing the cumulative incidence of first significant liver-related event (A) and survival without liver-related death (B) during follow-up.

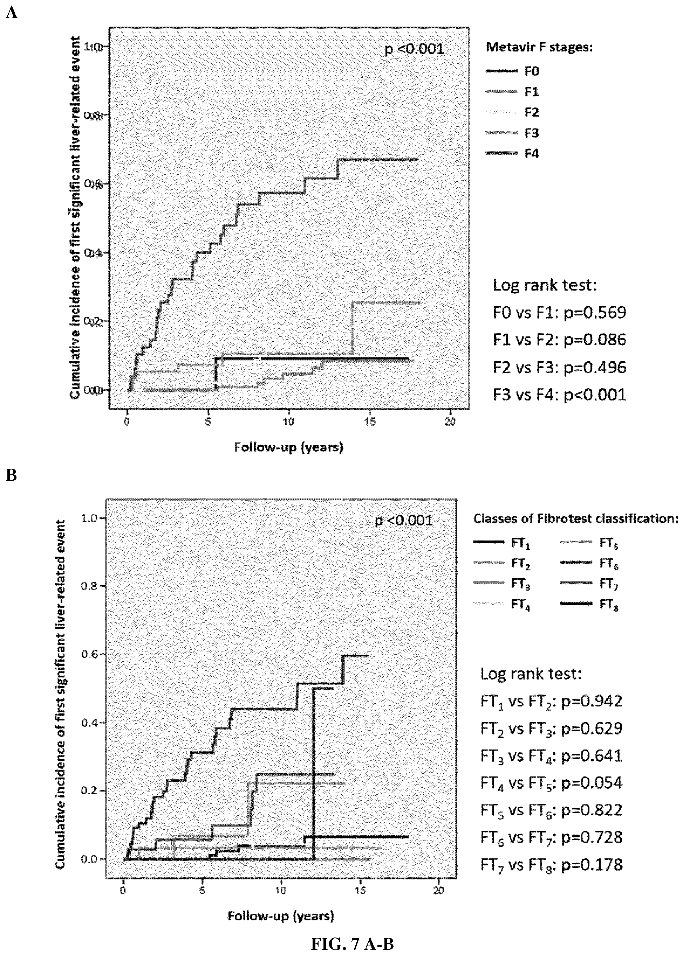

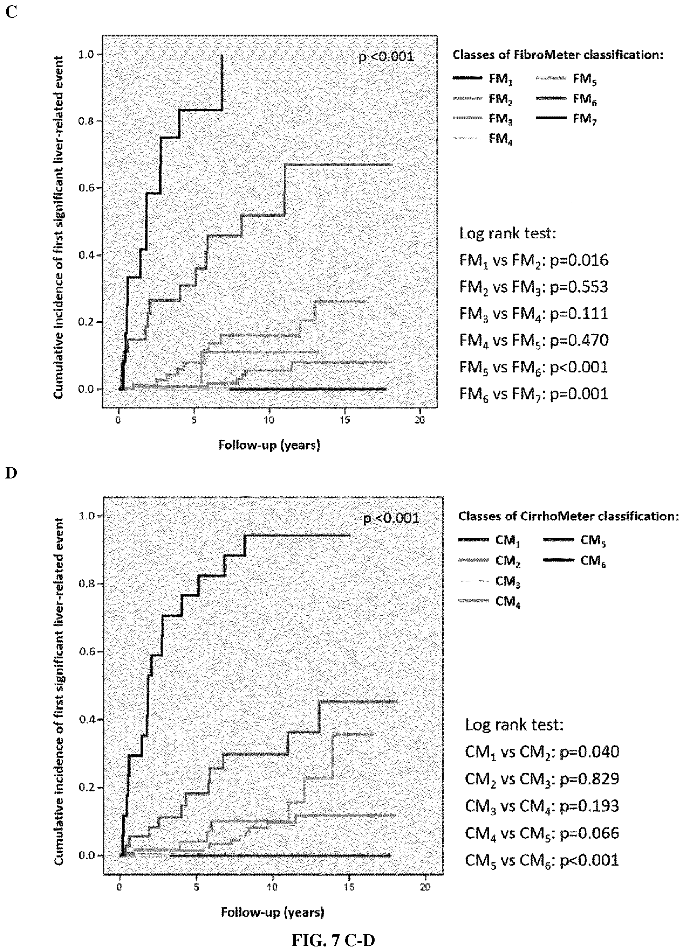

**Figure 7** is a combination of graphs showing the cumulative incidence of first significant liver-related events as a function of Metavir fibrosis (F) stages (A), or classes of Fibrotest (B), FibroMeter^{V2G} (C), CirrhoMeter^{V2G} (D) classifications. There was no significant difference among Metavir F0 to F3 stages (p=0.174) so that liver biopsy identified only 2 subgroups with different prognosis: F0-3 vs F4 (p<0.001). Diagnostic fibrosis classifications used in clinical practice to estimate the Metavir fibrosis stage from FibroMeter^{V2G} or CirrhoMeter^{V2G} results are also prognostic scales: unlike for Fibrotest, the increase in FibroMeter^{V2G} or CirrhoMeter^{V2G} classes was associated with a progressively worsening prognosis. The first upper curve corresponds to the higher fibrosis stage and so on.

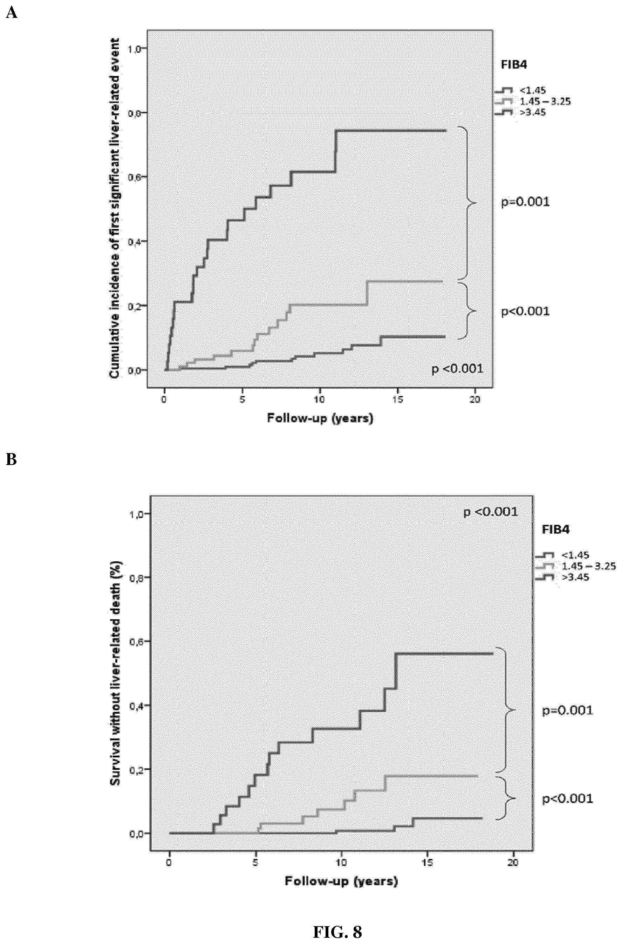

**Figure 8** is a combination of graphs showing the cumulative incidence of first significant liver-related event (A) and survival without liver related death (B) as a function of the 3 subgroups defined by the 2 recommended diagnostic cut-offs for FIB4 (< 1.45 and >3.25). The first upper curve corresponds to the higher fibrosis stage and so on.

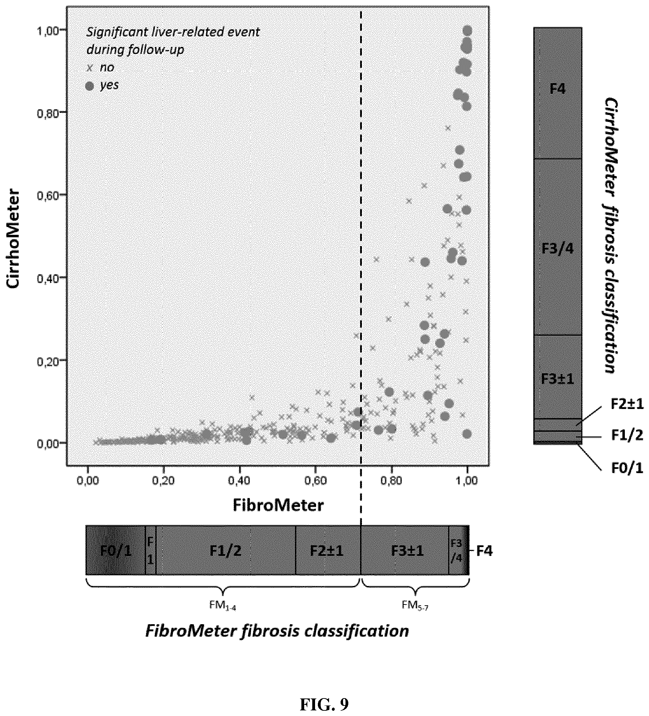

**Figure 9** is a graph showing the scatter plot of FibroMeter^{2GV} (dedicated to significant fibrosis diagnosis) and CirrhoMeter^{2GV} (for cirrhosis diagnosis), with their corresponding fibrosis classifications. These 2 blood tests are complementary with CirrhoMeter^{V2G} "stretching" high FibroMeter^{V2G} values. Two distinct groups can be distinguished: FM₁₋₄ classes displayed a larger range of FibroMeter^{V2G} values than CirrhoMeter^{V2G} values, and the opposite was observed in FM₅₋₇ classes.

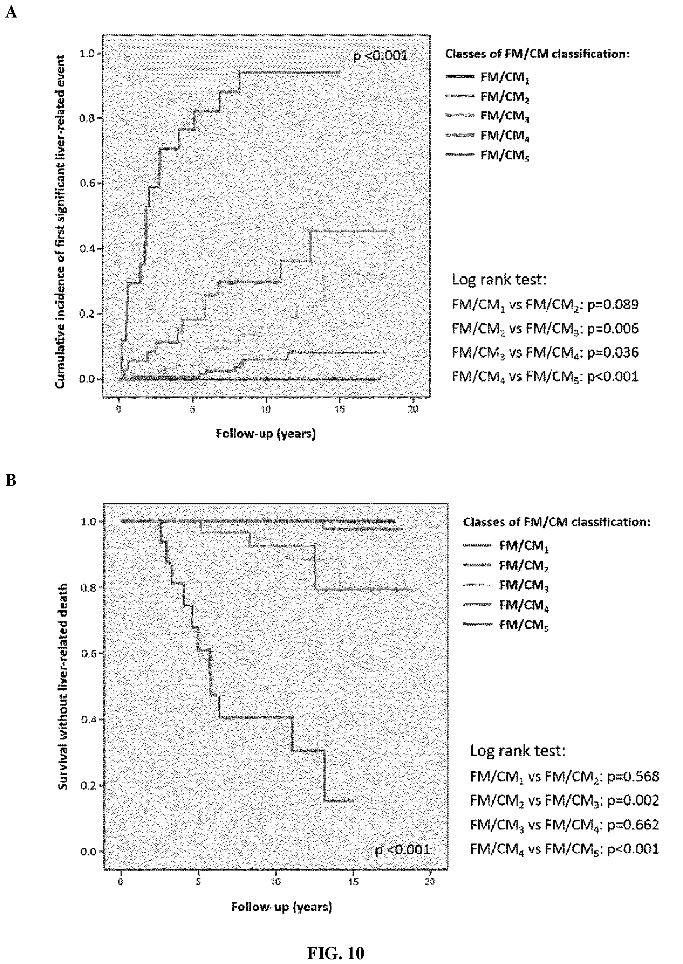

**Figure 10** is a combination of graphs showing the cumulative incidence of first significant liver-related events (A) and survival without liver-related death (B) as a function of the FM/CM classification. FM/CM classification identified 5 patient subgroups with significant different prognosis. A: The first upper curve corresponds to the higher fibrosis stage and so on. B: The first lower curve corresponds to the higher fibrosis stage and so on.

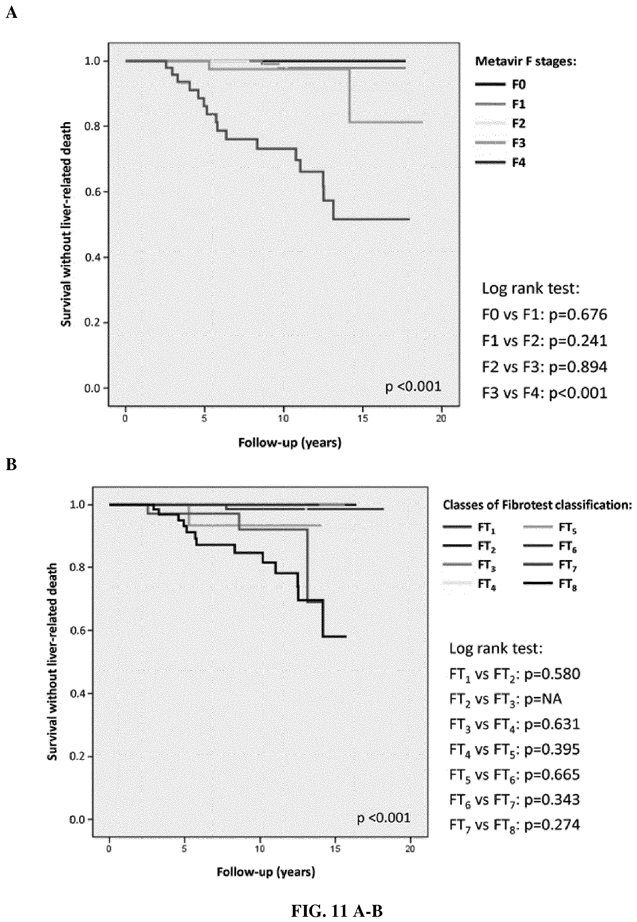

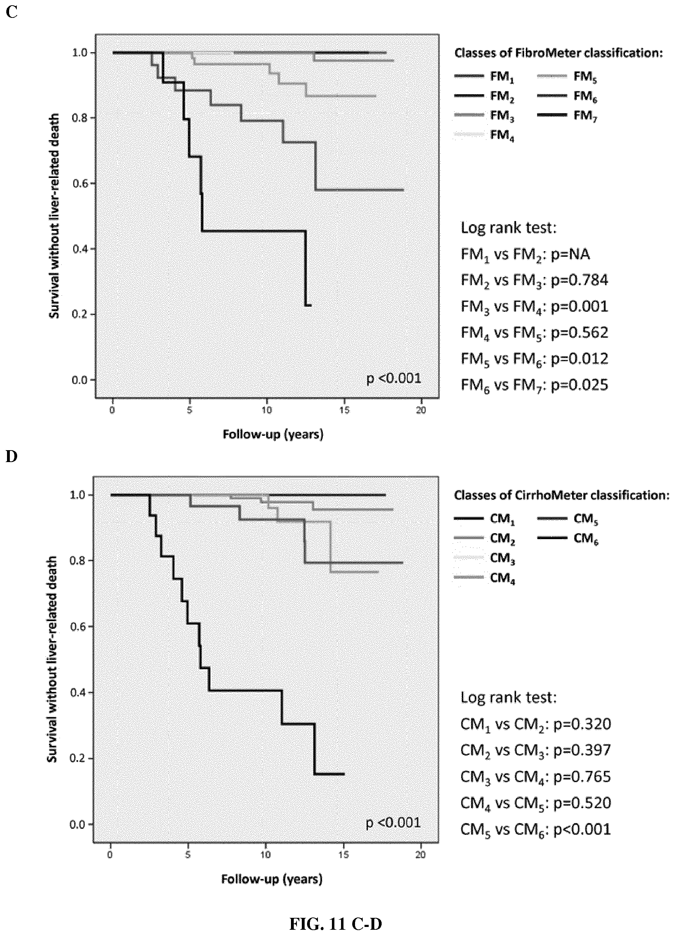

**Figure 11** is a combination of graphs showing the survival without liver-related death as a function of Metavir fibrosis (F) stages (A), or classes of Fibrotest (B), FibroMeter (C), CirrhoMeter (D) classifications. The first lower curve corresponds to the higher fibrosis stage and so on.

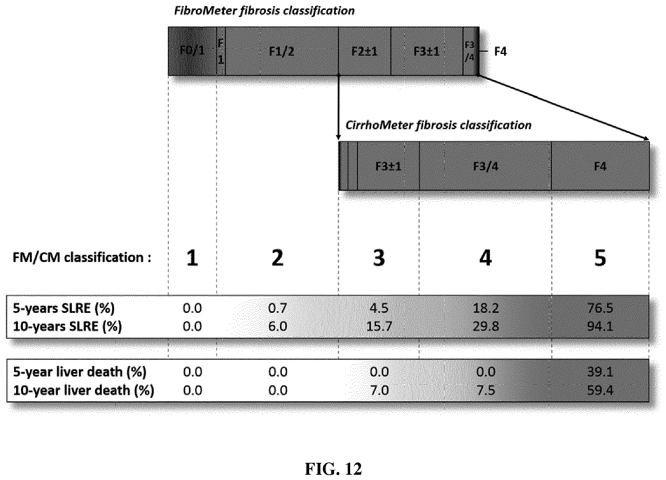

**Figure 12** is an image showing evaluation of liver-related prognosis from the results of the FibroMeter^{V2G} and CirrhoMeter^{V2G} diagnostic classifications. In patients with mild disease (i.e., included in the 3 first classes of the FibroMeter^{V2G} classification), the FibroMeter^{V2G} classification is used to identify 2 prognostic subgroups. In patients with advanced disease (i.e., included in the 4 last classes of the FibroMeter^{V2G} classification), the CirrhoMeter^{V2G} classification identify 3 additional prognostic subgroups. Incidence of liver-related events progressively increases as a function of these 5 prognostic subgroups which define the prognostic FM/CM classification.
**Figure 13** is a combination of risk charts of all-cause death (1 survival) before 3 years. LSM: F0-F1 <7 kPa ; F2-F3 7-13.9 kPa; F4 ≥ 14 kPa - FibroMeter^{V2G}: F0-F1 <0.3754 ; F2-F3 0.3754-0.8342 ; F4 ≥ 0.8343 - A-B: risk charts of all-cause death for males (A: aged of less than 55, B: aged of 55 years old or more), C-D: risk charts of all-cause death for females (C: aged of less than 55, D: aged of 55 years old or more).

### EXAMPLES

The present invention is further illustrated by the following examples.

### Example 1: Time-fixed and time-dependent multivariate prognostic models

### PATIENTS AND METHODS

### Study population

We included all subjects over 18 years of age who consulted or were hospitalized for a chronic liver disease in the Department of Hepatology of the University Hospital of Angers from January 2005 to December 2009, and who agreed to participate in the cohort SNIFF (Score Non Invasif de Fibrose du Foie). Patients have been included in the cohort whatever severity and etiology of chronic liver disease (HVC, HVB, alcohol abuse, non-alcoholic fatty liver, other causes). They were followed until death or up to January 1, 2011.

### Non-invasive evaluation of liver fibrosis

All patients had a non-invasive evaluation of liver fibrosis at the time of inclusion in the cohort and at every visit during follow-up, either by a blood test or a liver stiffness measurement, or both.

Blood tests for liver fibrosis - Blood sample analyses were centralized in the laboratory of our hospital. Biological parameters measured (alpha-2-macroglobulin, platelet count, aspartate aminotransferase, alanine aminotransferase, gamma-glutamyl transpeptidase, bilirubin, hyaluronic acid, prothrombin index, urea) allowed the computation of at least one of the following blood tests for liver fibrosis: APRI (Wai et al, Hepatology 2003; 38:518-26), FIB-4 (Sterling et al, Hepatology 2006; 43:1317-25), Hepascore (Adams et al, Clin Chem 2005; 51:1867-73), FibroMeter^{V2G} (Leroy et al, Clin Biochem 2008; 41:1368-76). The MELD score which has been specifically designed to predict short-term mortality in patients with end-stage of liver disease (Kamath et al, Hepatology 2001; 33(2):464-70) was also calculated in cirrhotic patients.

Liver stiffness measurement - Liver stiffness measurement (LSM) by Fibroscan (Echosens, Paris, France) was performed with the M probe. Examination conditions were those recommended by the manufacturer, with the objective of obtaining at least 10 valid measurements. Results were expressed as the median (kilopascals) of all valid measurements, and classified as "very reliable", "reliable" and "poorly reliable" by using ratio of interquartile range to median value (IQR/M) according to liver stiffness level: "very reliable" (IQR/M≤0.10), "reliable" (0.10<IQR/M≤0.30, or IQR/M>0.30 with M<7.1 kPa), "poorly reliable" (IQR/M>0.30 with M>7.1 kPa).

### Endpoint assessment

The endpoints of interest for these analyses were all-cause mortality and liver-related mortality. The date and primary cause of death were recorded during follow-up and checked in May 2011 using the computerized national death registry. Data for those patients who could not be found by this way were obtained from hospital database, and confirmed by information from general practitioner. Causes of death were coded according to the 10^{th} revision of the International Classification of Diseases (ICD-10) and pooled into categories.

### Statistical methods

Statistical analyses were performed using SAS version 9.2 (SAS Institute Inc., Cary, NC, USA) and R version 3.0.2 (Vienna, Austria, available from: http://www.R-project.org/). All tests were two-sided, and p<0.05 was considered statistically significant.

Data are expressed as the median (interquartile range), or frequency (%), as appropriate. Group comparisons were performed using Fisher's exact test for categorical data and nonparametric Mann-Whitney U test for continuous variables. Kaplan-Meier analyses with log-rank comparisons were used to calculate the median survival times and the survival differences between quartiles of each liver fibrosis diagnostic test at baseline.

Comparison of prognostic accuracy of non-invasive fibrosis tests - The predictive performance of non-invasive liver fibrosis tests was firstly evaluated on the entire follow-up by the Harrell's C-index. This non-parametric measure corresponds to an extension of the binary outcome AUC for time-to-event outcomes (censored survival data), and evaluates the amount of agreement between predictions (that are here the baseline values of fibrosis test) and the observed survival time comparing not only subjects with events to those without events, but also subjects having events that happened at different points in time. The results of C-index vary between 0.5 (no predictive power) and 1 (perfect concordance). The 95% confidence interval of each C-index was calculated by a bootstrap method, using 1,000 random samples (with replacement) of the same size as the original data set. Paired comparisons of C-indexes of fibrosis tests were also performed using a bootstrap resampling procedure. Briefly, the difference between two C-indexes was calculated on each of the 1,000 bootstrap samples to obtain the bootstrap 95% confidence interval of the difference. Then, the estimate of the standard error from this bootstrap distribution was used to calculate the standardized difference between the C-indexes and find the corresponding p-value.

Thereafter, we examined the ability of non-invasive fibrosis tests to predict the risk of death at various time horizons (different delays of prediction) from 0 to 5 years. The reported AUC(t) were computed and compared using the *timeROC* R package, they are based on the cumulative/dynamic ROC curve definition at a given time t and are corrected for right-censoring. Time-dependent ROC and corresponding AUC can be defined in different ways, the cumulative cases/dynamic controls definition is usually considered the most relevant as clinicians often want to discriminate subjects who have the event before time *t* (and not at a specific time *t,* as in "incident cases" definition) and subjects free of the event at time t (and not at a later fixed pre-specified time, e.g., the end of the study, as in "static controls" definition). The interpretation of cumulative/dynamic AUC(t) is similar to that of a standard AUC: the higher the AUC(t) is, the better the performance of the test to discriminate between patients who will do the event before time t and other patients.

Multivariate prognostic models for all-cause and liver-related mortality - We randomly split the sample of 1,565 patients having all tests into a training set (n=783) and a validation set (n=782). We built prognostic models with the training set. Age, sex, etiology of chronic liver disease and all non-invasive liver fibrosis tests were evaluated as potential predictors of death in both time-fixed and time-dependent multivariate Cox models. In the time-fixed model, only the baseline values of fibrosis tests were taken into account. The time-dependent model additionally used the repeated measurements of the liver fibrosis tests until 1 year before the end of follow-up. We applied the method of Murtaugh, et al. (Hepatology 1994; 20:126-34) to update the covariate over time in the model. We have assumed that the values observed on a patient at a given time remain unchanged until the next measurement because this corresponds to the clinical situation. By dividing each patient's follow-up in successive intervals and taking into account the measurements available at the start of each, 1337 survival data points were generated for the training set and 1342 for the validation set. For each multivariate model, assessment of proportional hazards was performed. We also tested the one-way interactions between the independent predictors, and checked that continuous covariates fit the appropriate functional form in the Cox model.

The accuracy of time-fixed and time-dependent prognostic models was evaluated in the validation set both in terms of calibration and discrimination performance. The calibration was assessed by comparing observed and estimated survival rates of the patients divided into 5 groups (quintiles) according to their estimated risk of death. Observed survival rates for each quintile group were calculated according to the Kaplan-Meier method. Estimated survival rates were computed as described elsewhere (Ripoll et al, Hepatology 2005; 42:793-801): mean values of each variable (most frequent modality for categorical variable) in the multivariate Cox model were calculated in each quintile subgroup and the predicted survival rate of the subgroup was defined as the death risk score calculated for these mean values (Σbᵢmᵢ, where bᵢ represents the estimated regression coefficient of each variable in model and mᵢ represents the mean value of the variable in the subgroup). Discrimination ability of prognostic models was assessed by the Harrell's C-index.

### RESULTS

### Characteristics of patients

A total of 3,352 patients have been enrolled in the cohort at the end of December 2009, their main baseline characteristics are summarized in **Table 1.** More than two thirds of subjects were males, the average age at inclusion was 54.9 ± 14.3. The chronic liver disease was due to excessive alcohol consumption in 42.7% of patients, whereas 30.4% had viral chronic hepatitis, 19.0% had non-alcoholic fatty liver disease (NAFLD) and the remaining 7.9% had other etiologies. A liver stiffness measurement was performed at baseline in 3,079 patients (91.9%), median baseline LSM result was 8.5 [1^{st} and 3^{rd} quartiles: 5.1 - 9.5]. Each liver fibrosis blood test was available in more than 2,500 patients (>75%) of the cohort.

**Table 1. Characteristics of the patients.**

| **Characteristics** | | | **Whole cohort** (n=3,352) | **All non-invasive liver fibrosis tests within a 3-month interval at inclusion** | | |
|---|---|---|---|---|---|---|
| | | | | **No** (n=1,787) | **Yes** (n=1,565) | ***P-value ^{(a)}*** |
| ***Baseline*** | | | | | | |
| Age (years) | | | 55.2 (45.2 ; 64.6) | 55.0 (45.8 ; 64.7) | 55.3 (44.4 ; 64.5) | *0.43* |
| Male sex (%) | | | 67.5 | 66.3 | 68.8 | *0.11* |
| Main cause of chronic liver disease (%) | | | 42.7 | 44.1 | 41.1 | *0.23* |
| | - Alcohol | | 30.4 | 30.4 | 30.5 | |
| | - Virus | | 19.0 | 18.1 | 20.0 | |
| | - NAFLD | | 7.9 | 7.4 | 8.4 | |
| | - Other | | | | | |
| LSM (kPa) (n=3,079) | | | | | | |
| | | median value | 8.5 | 7.9 | 8.9 | *0.0009* |
| | | interquartile | (5.5 ; 21.0) | (5.3 ; 18.6) | (5.6 ; 24.2) | *0.10* |
| range/median value | | | 0.17 (0.11 ; 0.25) | 0.17 (0.11 ; 0.25) | 0.17 (0.10 ; 0.25) | |
| APRI (n=2,615) | | | 0.40 (0.25 ; 0.84) | 0.42 (0.25 ; 0.80) | 0.39 (0.24 ; 0.86) | *0.35* |
| FIB-4 (n=2,607) | | | 1.65 (1.02; 3.38) | 1.78 (1.14; 3.41) | 1.58 (0.94 ; 3.37) | *0.0003* |
| Hepascore (n=2,588) | | | 0.59 (0.22 ; 0.99) | 0.69 (0.28 ; 1.00) | 0.54 (0.20 ; 0.99) | *<0.0001* |
| FibroMeter^{V2G} (n=2,523) | | | 0.61 (0.28 ; 0.93) | 0.65 (0.34 ; 0.93) | 0.58 (0.24 ; 0.93) | *0.0007* |

| ***Follow-up*** | | | | | | |
|---|---|---|---|---|---|---|
| Follow-up time (years) | | | 3.3 (1.9; 4.6) | 3.8 (2.2; 5.2) | 2.8 (1.7 ; 3.9) | *<0.0001* |
| Outcomes | | | | | | |
| | - All-cause death (%) | | 17.5 | 18.2 | 16.8 | *0.29* |
| | - Liver-related death (%) | | 7.7 | 7.8 | 7.4 | *0.57* |
| Results for quantitative variables are expressed as median (1^{st} quartile; 3^{rd} quartile). | | | | | | |
| LSM=Liver stiffness measurement, NAFLD=Non-alcoholic fatty liver disease. | | | | | | |

| | | | | | | |
|---|---|---|---|---|---|---|
| (a) p-value from Fisher's exact test (categorical variables) or Mann-Whitney-Wilcoxon test (quantitative variables). | | | | | | |

On 1st January 2011, the median duration of follow-up in the whole cohort was 3.3 years [interquartile range (IQR) 1.9-4.6]. During follow-up period, 588 patients (17.5%) died, 258 deaths (7.7%) were liver-related. Kaplan-Meier estimates of overall survival (without all-cause death) at 1, 3 and 5 years were respectively 91.6%, 83.0% and 75.5%. The liver-related survival (without liver-related death) at 1, 3 and 5 years were 95.7%, 92.5% and 86.6%.

According to data from the French national death registry, the other causes of death were mainly neoplasms unrelated to liver and intrahepatic bile ducts (ICD-10 C00-D48 except C22, n=104), diseases of the circulatory system (ICD-10 I00-I99, n=79), external causes of morbidity and mortality (ICD-10 V01-Y98, n=36), endocrine nutritional and metabolic diseases (ICD-10 E00-E90, n=17), diseases of the digestive system other than of the liver (ICD-10 K00-K93 except K70-K77, n=16). Cause of death was unknown in 12 patients.

### Univariate relationships with mortality

Patients who died had quite different baseline characteristics, as compared to those alive at the end of follow-up **(Table 2):** they were older and more frequently males, they were twice to have an alcoholic liver disease and had more severe disease at baseline, as reflected of all non-invasive liver fibrosis tests at baseline. Kaplan-Meier estimates of both overall survival and liver-related survival significantly differed according to quartiles of each liver fibrosis test values at baseline (all p-values from log-rank comparisons <0.0001).

**Table 2. Comparison of baseline characteristics according to vital status at the end of follow-up in the whole cohort.**

| **Characteristics** | | **N** | **Vital status at the end of follow-up** | | |
|---|---|---|---|---|---|
| | | | **Alive** (n=2764) | **Dead** ^{(a)} (n=588) | ***P-value***^{(b)} |
| Age (years) | | 3352 | 53.2 (43.9 ; 62.2) | 62.8 (54.9 ; 73.6) | *<0.0001* |
| Male sex (%) | | 3352 | 65.2 | 77.9 | *<0.0001* |
| Main cause of chronic liver disease (%) | | 3352 | 37.1 | 69.1 | *<0.0001* |
| | - Alcohol | | 33.8 | 14.8 | |
| | - Virus | | 20.3 | 12.9 | |
| | - NAFLD | | 8.8 | 3.2 | |
| | - Other | | | | |
| AST (IU/L) | | 2615 | 34 (24; 54) | 49 (30 ; 81) | *<0.0001* |
| ALT (IU/L) | | 2607 | 35 (23 ; 59) | 32 (21 ; 52) | *0.0027* |
| GGT (IU/L) | | 2588 | 65 (31 ; 164) | 155 (73 ; 337) | *<0.0001* |
| Bilirubin (µmol/L) | | 2594 | 9 (6 ; 15) | 18 (10 ; 40) | *<0.0001* |
| Urea (µmol//L) | | 2523 | 4.9 (3.9 ; 6.1) | 4.9 (3.4 ; 7.0) | *0.89* |
| Platelets (g/L) | | 2647 | 209 (157 ; 261) | 152 (102 ; 226) | *<0.0001* |
| Prothrombin time (%) | | 2651 | 93 (83 ; 103) | 74 (56; 87) | *<0.0001* |
| Alpha 2 macroglobulin (mg/dL) | | 2645 | 227 (173 ; 312) | 256 (195 ; 312) | *<0.0001* |
| Hyaluronic acid (µg/L) | | 2651 | 54 (28 ; 138) | 269 (110; 621) | *<0.0001* |
| APRI | | 2615 | 0.37 (0.24; 0.71) | 0.76 (0.35 ; 1.62) | *<0.0001* |
| FIB-4 | | 2607 | 1.47 (0.94 ; 2.65) | 3.83 (1.95 ; 6.68) | *<0.0001* |
| Hepascore | | 2588 | 0.48 (0.19; 0.94) | 1.00 (0.85; 1.00) | *<0.0001* |
| FibroMeter^{V2G} | | 2523 | 0.52 (0.24 ; 0.85) | 0.96 (0.82 ; 1.00) | *<0.0001* |
| LSM (kPa) | | | | | |
| | all results | 3079 | 7.6 (5.3 ; 14.8) | 27.3 (10.1 ; 63.9) | *<0.0001* |
| | very reliable/reliable results ^{(c)} | 2740 | 6.8 (5.1 ; 12.9) | 27.4 (8.7 ; 69.1) | *<0.0001* |

| | | | | | |
|---|---|---|---|---|---|
| Quantitative variables are expressed as median (1^{st} quartile ; 3^{rd} quartile). LSM=Liver stiffness measurement. (a) All-cause death. (b) P-value from Fisher's exact test (categorical variables) or Mann-Whitney-Wilcoxon test (quantitative variables). (c) As classified by recently proposed reliability criteria: "very reliable" (IQR/median ≤ 0.10) and "reliable" (0.10<IQR/median≤0.30, or IQR/median>0.30 with LSE median<7.1 kPa). | | | | | |

### Comparisons of the ability of liver fibrosis tests to predict mortality

Statistical comparisons of the predictive power of non-invasive liver fibrosis tests were performed in the subsample of patients having available data for all tests within a 3-month interval at inclusion. There was no difference in socio-demographic characteristics and causes of chronic liver disease between these 1,565 patients as compared to the other patients from the cohort **(Table 1).** But patients having all tests were more recently included in the cohort, and consequently had a slightly lower follow-up duration: median follow-up was 2.8 years [1^{st} and 3^{rd} quartiles: 1.7 - 3.9]. They had also a higher baseline liver stiffness median value than non-selected patients, but conversely they had lower baseline values for most liver fibrosis blood tests.

The global predictive power of APRI, as assessed by the Harrell's C-index, was much lower than other non-invasive liver fibrosis tests for both all-cause mortality and liver-related mortality **(Table 3).**

**Table 3. Overall discriminative ability of mortality risk prediction by non-invasive liver fibrosis tests.**

| | **Whole cohort** (n=3,352) | | **All non-invasive liver fibrosis tests** |
|---|---|---|---|
| | | | **within a 3-month interval at inclusion** (n=1,565) |
| | **n** | **Harrell's C-index [95 % CI]** | **Harrell's C-index [95% CI]** |
| **All-cause mortality** | | | |
| LSM | 3079 | 0.745 [0.722-0.768] | 0.758 [0.725-0.790] |
| LSM-r | 2740 | 0.749 [0.723-0.776] | 0.766 [0.730-0.801] |
| APRI | 2615 | 0.655 [0.626-0.683] | 0.661 [0.625-0.697] ^{(a)} |
| FIB-4 | 2607 | 0.744 [0.721-0.767] | 0.753 [0.725-0.784] |
| Hepascore | 2588 | 0.761 [0.738-0.783] | 0.771 [0.741-0.800] |
| FibroMeter^{V2G} | 2523 | 0.781 [0.759-0.800] | 0.792 [0.765-0.817] ^{(b)} |

| **Liver-related mortality** | | | |
|---|---|---|---|
| LSM | 3069 | 0.867 [0.842-0.890] | 0.872 [0.838-0.901] |
| LSM-r | 2731 | 0.879 [0.853-0.906] | 0.891 [0.855-0.924] |
| APRI | 2605 | 0.765 [0.733-0.799] | 0.774 [0.730-0.817] ^{(c)} |
| FIB-4 | 2597 | 0.833 [0.809-0.858] | 0.843 [0.812-0.875] ^{(d)} |
| Hepascore | 2578 | 0.867 [0.844-0.886] | 0.878 [0.851-0.905] |
| FibroMeter^{V2G} | 2513 | 0.876 [0.857-0.894] | 0.882 [0.857-0.906] |

| | | | |
|---|---|---|---|
| 95% CI and differences in C-index were assessed by a bootstrap procedure. LSM=Liver stiffness measurement, LSM-r=LSM results classified as very reliable/reliable for diagnostic purpose. **(a)** significantly lower than LSM (p<0.0001), FIB-4 (p<0.0001), Hepascore (p<0.0001), FibroMeter^{V2G} (p<0.0001). **(b)** significantly higher than those of LSM (p<0.040), APRI (p<0.0001), FIB-4 (p<0.0001), Hepascore (p=0.0086). **(c)** significantly lower than LSM (p<0.0001), FIB-4 (p<0.0001), Hepascore (p<0.0001), FibroMeter^{V2G} (p<0.0001). **(d)** significantly lower than FibroMeter^{V2G} (p<0.0001) and LSM very reliable/reliable results (p<0.030). | | | |

FibroMeter^{V2G} had the greatest performance in predicting all-cause mortality, significant difference was observed as compared to all other tests. The global prognostic performance of LSM was only marginally altered when including LSM results considered as poorly reliable for diagnostic purpose. For liver-related mortality, both FibroMeter^{V2G} and LSM had higher prognostic value, with significant differences in comparison with APRI and FIB-4. According to time-dependent AUC, liver fibrosis tests had similar trend of predictive accuracy over time: as expected, the predictive power of tests was the greatest in the few months after the measure, then decreased to a performance level that remains relatively stable until 5 years **(****Figure 1****).** To summarize, AUC(t)>0.70 for all-cause mortality, AUC(t) from 0.80 to 0.90 for liver-related mortality. As example, for predicting 3-year death from all causes, AUC(t=3) were: 0.80 (95% CI=[0.76-0.83]) for FibroMeter^{v2G}, 0.77 [0.74-0.81] for Hepascore (p<0.001 vs FibroMeter^{V2G)}, 0.76 [0.73-0.80] for LSM (p<0.028), 0.76 [0.72-0.79] for FIB4 (p<0.001) and 0.67 [0.62-0.71] for APRI (p<0.001).

We further compared the AUC(t) of liver fibrosis tests to MELD score in the subsample of 448 patients having cirrhosis, as defined by LSM ≥ 14 KPa. Only 4 cirrhotic patients had not available data for MELD at baseline, results of the time-dependent ROC curve analysis in the remaining 444 patients are graphically presented in **Figure 2****.** In cirrhotic patients, the MELD score had higher prognostic ability than Hepascore and FIB-4 for predicting death up to 2 years following initial non-invasive measures, and was significantly better than LSM for deaths that occurred in the first 18 months. In contrast, the differences in AUC(t) between FibroMeter^{V2G} and MELD were not statistically significant. For prediction of liver-related mortality, both FibroMeter^{V2G} and LSM did not significantly differ from MELD at any time point. As summarized by the Harrell's C-index, APRI and FIB-4 were less efficient than FibroMeter^{V2G} (vs APRI: p=0.0003, vs FIB-4: p=0.026), and LSM (vs APRI: p=0.026) in discriminating cirrhotic patients according to their risk of liver-related death.

Comparative analysis between liver fibrosis tests were also performed in the 3 main causes of chronic liver disease separately (alcoholic liver disease (ALD), chronic viral hepatitis, NAFLD). Results of time-dependent AUC are reported in **Figure 3****.** In patients having ALD, FibroMeter^{V2G} showed better performance (AUC(t)>0.70 with t varying from 0 to near 5 years) than other liver fibrosis tests for all-cause death prediction. To summarize, all paired comparisons of C-indexes between FibroMeter^{V2G} and other liver fibrosis led to p<0.05. For liver-related death, only the C-index difference between FibroMeter^{V2G} and Hepascore was not statistically significant. In patients having chronic viral hepatitis and NAFLD, all liver fibrosis tests (except APRI) had a good discriminative ability (>0.80) for liver-related death, whereas there were more variations in AUC(t) over time for all-cause death. In particular, the prognostic value of baseline measure of tests slightly decreased with increasing delay of prediction in patients with NALFD.

### Multivariate prognostic models based only on baseline liver fibrosis tests

Multivariate analyses were performed after randomly splitting the subsample of 1,565 patients having all tests at baseline into a training set and a validation set. The main characteristics of these two subgroups of patients are presented in **Table 4.**

**Table 4. Comparison of patient characteristics between randomly selected training and validation sets.**

| **Characteristics** | | **Training set** (n=783) | **Validation set** (n=782) | ***P-value*** |
|---|---|---|---|---|
| ***Baseline*** | | | | |
| Age (years) | | 55.6 (44.4 ; 65.6) | 55.0 (44.7 ; 63.0) | *0.53* |
| Male sex (%) | | 67.8 | 69.8 | *0.41* |
| Main cause of chronic liver disease (%) | | 41.1 | 41.0 | *0.082* |
| | - Alcohol | 29.9 | 31.2 | |
| | - Virus | 18.9 | 21.1 | |
| | - NAFLD | 10.1 | 6.7 | |
| | - Other | | | |
| AST (IU/L) | | 37 (24 ; 62) | 35 (25 ; 59) | *0.71* |
| ALT (IU/L) | | 35 (22 ; 61) | 36 (23 ; 58) | *0.97* |
| GGT (IU/L) | | 80 (33 ; 208) | 81 (35 ; 207) | *0.88* |
| Bilirubin (µmol/L) | | 10 (6 ; 18) | 10 (7 ; 18) | 0.52 |
| Urea (µmol/L) | | 4.9 (3.7 ; 6.1) | 4.8 (3.6 ; 6.1) | *0.76* |
| Platelets (g/L) | | 207 (146 ; 266) | 209 (155 ; 263) | *0.68* |
| Prothrombin time (%) | | 92 (77 ; 103) | 91 (78 ; 101) | *0.54* |
| Alpha 2 macroglobulin (mg/dL) | | 221 (171 ; 298) | 216 (168 ; 294) | *0.45* |
| Hyaluronic acid (µg/L) | | 61 (29 ; 207) | 61 (28 ; 188) | 0.77 |
| APRI | | 0.40 (0.24 ; 0.90) | 0.39 (0.25 ; 0.82) | *0.73* |
| FIB-4 | | 1.61 (0.94; 3.51) | 1.56 (0.94; 3.14) | *0.57* |
| Hepascore | | 0.54 (0.19 ; 0.99) | 0.53 (0.20 ; 0.99) | *0.96* |
| FibroMeter^{V2G} | | 0.61 (0.24 ; 0.93) | 0.56 (0.25 ; 0.92) | *0.71* |
| LSM (kPa) | | 8.8 (5.6 ; 26.0) | 8.9 (5.6 ; 22.0) | *0.88* |

| ***Follow-up*** | | | | |
|---|---|---|---|---|
| Follow-up time (years) | | 2.7 (1.7 ; 3.9) | 2.8 (1.7 ; 4.0) | *0.72* |
| Outcomes | | | | |
| | - All-cause death (%) | 16.6 | 17.0 | *0.84* |
| | - Liver-related death (%) | 7.3 | 7.6 | 0.85 |

By multivariate analysis in the training set, we observed that each blood fibrosis test and LSM were independent prognostic factors (data not shown). Age and sex were also significant in all multivariate Cox models, but not the cause of chronic liver disease, nor the interquartile range of LSM results. According to the Akaike information criteria (AIC), the best multivariate Cox model was the one that included age, sex, LSM and FibroMeter^{v2G}, either to predict all-cause mortality or mortality due liver disease. No interactions between the independent predictors were found. Details of the coefficients of the multivariate models minimizing the AIC are given in **Table 5.**

**Table 5. Best multivariate Cox models minimizing AIC to predict mortality based on time-fixed approach (A) and on time-dependent approach (B).**

| | | **Parameter** | **Standard Error** | ***P-value*** |
|---|---|---|---|---|
| **Time-fixed Cox model** | | | | |
| | **A. All-cause mortality** | | | |
| | Sex (reference=men) | -0.72764 | 0.23102 | 0.0016 |
| | Age | 0.04904 | 0.00741 | <0.0001 |
| | LSM | 0.01271 | 0.00460 | 0.0057 |
| | FibroMeter^{V2G 10} | 1.55638 | 0.30547 | <0.0001 |

| | **B. Liver-related mortality** | | | |
|---|---|---|---|---|
| | Sex (reference=men) | -1.19155 | 0.43356 | 0.0060 |
| | Age | 0.04153 | 0.01230 | 0.0007 |
| | LSM | 0.02104 | 0.00658 | 0.0014 |

| | | **Parameter** | **Standard Error** | ***P-value*** |
|---|---|---|---|---|
| **Time-fixed Cox model** | | | | |
| | **A. All-cause mortality** | | | |
| | FibroMeter^{V2G} | 12.74142 | 3.23933 | <0.0001 |

| **Time-dependent Cox mode** | | | | |
|---|---|---|---|---|
| | **A. All-cause mortality** | | | |
| | Sex (reference=men) | -0.70222 | 0.23183 | 0.0025 |
| | Age | 0.04920 | 0.00737 | <0.0001 |
| | LSM | 0.01353 | 0.00459 | 0.0032 |
| | FibroMeter^{V2G 30} | 1.70878 | 0.28575 | <0.0001 |

| | **B. Liver-related mortality** | | | |
|---|---|---|---|---|
| | Sex (reference=men) | -1.14706 | 0.43453 | 0.0083 |
| | Age | 0.03913 | 0.01216 | 0.0013 |
| | LSM | 0.02127 | 0.00694 | 0.0022 |
| | FibroMeter^{V2G 10} | 3.79448 | 0.63567 | <0.0001 |

**Figure 4A** depicts the overall survival curve predicted by the prognostic model (derived from the training set) in patients from the validation set who were stratified according to their risk score into 5 groups (quintiles). We observed a good fit between the predicted and observed overall survival: the predicted overall survival rates at 3 years were 98.6%, 94.3%, 90.6%, 80.5% and 46.7% from quintile 1 to quintile 5, whereas the observed overall survival rates were 99.0%, 97.6%, 93.0%, 78.0% and 50.9% respectively. In addition, the prognostic model combining LSM and FibroMeter^{V2G} had an improved discriminative ability: the Harrell's C-index [95% CI] of the prognostic model in the validation set was 0.834 [0.805-0.863], as compared to 0.791 [0.756-0.826] for FibroMeter^{V2G} solely (p<0.0001), and 0.775 [0.736-0.811] for LSM (p=0.0013). The prognostic multivariate model had better performance than FibroMeter^{V2G} and LSM in ALD, and it was more efficient than FibroMeter^{V2G} but not than LSM in chronic viral hepatitis **(Table 6,** details regarding paired comparisons not shown).

**Table 6. Discriminative ability, as assessed by Harrell's C-index [95% CI], of multivariate prognostic models including age, sex, LSM and FibroMeter^{v2G}, in the validation set (n=782).**

| | **Time-fixed prognostic models** | | **Time-dependent prognostic models** | |
|---|---|---|---|---|
| | **All-cause mortality** | **Liver-related mortality** | **All-cause mortality** | **Liver-related mortality** |
| Overall | 0.834 [0.805-0.863] | 0.867 [0.828-0.902] | 0.837 [0.808-0.865] | 0.879 [0.840-0.914] |

| **According to cause of chronic liver disease** | | | | |
|---|---|---|---|---|
| Alcohol | 0.742 [0.691-0.790] | 0.798 [0.732-0.858] | 0.754 [0.704-0.799] | 0.832 [0.774-0.881] |
| Virus | 0.870 [0.780-0.939] | 0.919 [0.856-0.970] | 0.857 [0.752-0.928] | 0.918 [0.862-0.966] |
| NAFLD | 0.840 [0.734-0.927] | 0.861 [0.735-0.969] | 0.849 [0.757-0.935] | 0.856 [0.706-0.961] |

| | | | | |
|---|---|---|---|---|
| 95% CI of Harrell were assessed by a bootstrap procedure. LSM=Liver stiffness measurement. NAFLD=Non-alcoholic fatty liver disease. | | | | |

The agreement between the observed and predicted survival (calibration) was less satisfying **(****Figure 4B****):** the probabilities of liver-related survival beyond 1 year were underestimated in patients from the highest risk quintile, while the survival rates of patients having a slightly lower risk score (from the 4th quintile) were overestimated. In addition, there was no gain in accuracy by applying the prognostic model to the validation set, as compared to using of a single non-invasive liver fibrosis test: the Harrell's C-index [95% CI] of the prognostic model was 0.867 [0.828-0.902], as compared to 0.857 [0.815-0.895] using only FibroMeter^{V2G} (p=0.27), and 0.848 [0.785-0.896] using LSM (p=0.43).

### Multivariate prognostic models with liver fibrosis tests defined as time-varying covariates

The non-invasive tests were carried out from 1 to 8 times from inclusion in the cohort to one year before the end of follow-up. The median interval length between measurements was 1.1 years [1^{st} and 3^{rd} quartiles: 0.6 - 1.7]. After applying the time-dependent prognostic model to the validation set, we observed a better match between predicted and observed liver-related survival **(****Figure 5****).** For all-cause and liver-related mortality, prognostic models with updating covariates were also based on age, sex, LSM and FibroMeter^{V2G} The Harrell's C-indexes and 95 % CI of these time-dependent multivariate Cox models were in the validation set were 0.837 [0.808-0.865] for prediction of all-cause death and 0.879 [0.840-0.914] for prediction of liver-related death **(Table 6).** In patients with ALD, the C-index increased to 0.832 [0.774-0.881] for the prediction of liver-related death.

We then compared overall prognostic performance though discriminative ability of liver-fibrosis diagnostic tests and of the prognostic model of the present invention, based on age, sex, LSM and prothrombin index, bilirubin, urea, hyaluronic acid. Prognostic performance was assessed by Harrell's index [95% CI] in the 1565 patients having all non-invasive liver fibrosis tests at inclusion in the cohort. Results are shown in **Table 7.**

**Table 7: Harrell's C index of liver-fibrosis diagnostic tests and of the prognostic model of the invention**

| | | **Prognostic target** | |
|---|---|---|---|
| | | **All-cause mortality** | **Liver-related mortality** |
| **Liver-fibrosis diagnostic tests** | LSM | 0.758 [0.726-0.788] ^{(a)} | 0.872 [0.839-0.902] ^{(d)} |
| | LSM-r | 0.766 [0.730-0.801] ^{(b)} | 0.891 [0.855-0.924] |
| | FibroMeter^{V2G} | 0.792 [0.767-0.818] | 0.882 [0.855-0.906] |
| | CirrhoMeter^{V2G} | 0.781 [0.754-0.808] | 0.870 [0.841-0.895] |
| | EFM^{V2G} | 0.777 [0.749-0.803] | 0.886 [0.863-0.908] |
| | FibroMeter^{V3G} | 0.780 [0.753-0.805] | 0.857 [0.827-0.883] |
| | CirrhoMeter^{V3G} | 0.776 [0.748-0.800] | 0.847 [0.819-0.874] |
| | EFM^{V3G} | 0.775 [0.746-0.800] | 0.881 [0.858-0.903] |
| **Prognostic score of the invention** | Risk score for predicting all-cause mortality | 0.826 ^{(c)} [0.803-0.848] | - |
| | Risk score for predicting liver-related mortality | - | 0.893 [0.869-0.915] ^{(e)} |

| | | | |
|---|---|---|---|
| 95% CI and differences in C-index were assessed by a bootstrap procedure. LSM=Liver stiffness measurement, LSM-r=LSM results classified as very reliable/reliable for diagnostic purpose. **(a)** significantly lower than those of FibroMeter^{V2G} (p<0.007), EFM^{V2G} (p<0.002), EFM^{v3G} (p<0.008) **(b)** significantly lower than those of FibroMeter^{V2G} (p<0.040) **(c)** significantly higher than all liver fibrosis diagnostic tests (p<0.001) **(d)** significantly lower than those of EFM^{V2G} (p<0.030) **(e)** significantly higher than CirrhoMeter^{V2G} (p<0.012), FibroMeter^{V3G} (p<0.001), CirrhoMeter^{V3G} (p<0.001) | | | |

These results provide new insight about the prognostic value of liver fibrosis diagnostic tests, comparing their ability to identify patients with higher risk of death, at various time points, during the 5 years following the initial evaluation of liver fibrosis. Furthermore, our results indicate that combining FibroMeter^{V2G} and LSM increased prognostic performance, and more particularly in patients with ALD where the prognostic value of LSM was lower than in other causes of chronic liver diseases.

### Example 2: Liver-prognosis assessment in chronic hepatitis C (CHC) patients

The first aim of this study was to compare the prognostic accuracy of several blood fibrosis tests and liver biopsy for the prediction of liver-related events in a large cohort of CHC patients with long term follow-up. Moreover, the second aim was to evaluate if a combination of blood fibrosis tests can improve the liver-prognosis assessment.

### PATIENTS AND METHODS

### Patients

We used a previously published database of CHC patients with liver biopsy and blood fibrosis tests from the University Hospital of Angers, France (tertiary referral center) (Calès et al, Liver Int 2008; 28:1352-62). Patients were included from 1994 to 2007 if they had CHC, defined as both positive anti-hepatitis C virus antibodies and hepatitis C virus RNA in serum. Exclusion criteria were other causes of chronic hepatitis (hepatitis B or HIV co-infection, alcohol consumption >30g/d in men or >20g/d in women in the 5 years before inclusion, hemochromatosis, autoimmune hepatitis), or cirrhosis complications (ascites, variceal bleeding, systemic infection, hepatocellular carcinoma). Study protocol conformed to the ethical guidelines of the current Declaration of Helsinki and all patients gave informed consent following an IRB approval.

### Data collection during follow-up

Follow-up started the day of liver biopsy and ended January 1^{st}, 2011. Patients were followed and received antiviral therapy according to current EASL and AASLD guidelines. Data recorded during follow-up included: achievement of SVR, date of first significant liver-related event, and date and cause of death. SVR was defined as negative hepatitis C virus RNA 6 months after the end of antiviral treatment. The significant liver-related events (SLRE) under consideration were those requiring specific therapy or care: ascites, encephalopathy, jaundice (serum bilirubin >50 µmol/l), occurrence of large esophageal varices (diameter ≥5 mm), variceal bleeding, hepatorenal syndrome, and hepatocellular carcinoma. Large esophageal varices were considered as significant liver-related event because their occurrence significantly impacts treatment and prognosis. Follow-up data were recorded in patient files; for cases lost to hospital follow-up, the patients or their general practitioners were called for follow-up data. Date and cause of death were obtained for all patients by consulting the national death registry. Transplanted patients were censored as dead at the date of liver transplantation. Finally, 2 clinical outcomes were evaluated in the present study: SLRE as primary endpoint (patients were censored at the date of the first event) and liver-related death as secondary endpoint.

### Histological assessment

Liver fibrosis was evaluated according to Metavir fibrosis (F) staging. Initial pathological examinations were independently performed by two senior experts specialized in hepatology, with a consensus reading for discordant cases. The pathologists were blinded for patient characteristics and blood marker results.

### Blood tests

Blood collection was performed within the 7 days around the date of liver biopsy in 87.4% of the cases, and no more than 3 months thereafter in the other cases. All blood assays were performed in the laboratory of Angers hospital.

***Blood fibrosis tests* -** The following blood fibrosis tests were calculated according to published or patented formulas: APRI (Wai et al, Hepatology 2003; 38:518-26), FIB4 (Sterling et al, Hepatology 2006; 43:1317-25), Hepascore (Adams et al, Clin Chem 2005; 51:1867-73), Fibrotest (Biopredictive, Paris, France) (Castera et al, Gastroenterology 2005; 128:343-50), FibroMeter^{V2G} (2^{nd} generation for virus; Echosens, Paris, France) (Leroy et al, Clin Biochem 2008; 41:1368-76). We also calculated CirrhoMeter^{V2G} (Echosens, Paris, France), which shares the same markers as FibroMeter^{V2G} but with specific coefficients targeted for the diagnosis of cirrhosis (Boursier et al, Eur J Gastroenterol Hepatol 2009; 21:28-38). In clinical practice, the results of some blood fibrosis tests are interpreted by using their *fibrosis stage classifications* that estimate Metavir F stage(s) from the test results. Fibrotest classification includes 8 classes (called here FT₁ to FT₈), FibroMeter^{V2G} includes 7 classes (FM₁ to FM₇), and CirrhoMeter^{V2G} 6 classes (CM₁ to CM₆).

### Statistical analysis

Survival curves were determined using the Kaplan-Meier method and compared with the log rank test. Discriminative ability for the prediction of clinical outcomes was evaluated by the C-index of Harrell. The C-index of Harrell is an extension of the AUROC for time-to-event (survival) data and evaluates the concordance between the predicted risk of event and the observed survival time. Its results varies from 0 to 1: 1 shows a perfect concordance (discriminative power of the risk score), 0.5 shows random prediction, and a value less than 0.5 indicates discrimination in the opposite direction to that expected. For each test evaluated, the 95% confidence interval of the Harrell C-index was calculated by a bootstrap method, using 1,000 random samples (with replacement) of the same size as the original data set. Paired comparisons of the C-indexes were performed using a bootstrap resampling procedure. To compare 2 tests, we calculated the difference between their C-indexes on each of the 1,000 random samples chosen with replacement from the original dataset. Then, the bootstrap 95% confidence interval of the difference was obtained, and we used the estimate of the standard error from this bootstrap distribution to calculate the standardized difference between the C-Indexes and find the corresponding p-value. Most statistical analyses were performed using SPSS version 18.0 software (IBM, Armonk, NY, USA) and SAS 9.1 (SAS Institute Inc., Cary, NC, USA) by a professional statistician (SB).

### RESULTS

### Patients

The main baseline characteristics of the 373 included patients are detailed in **Table 8.**

**Table 8: Patient characteristics at inclusion**

| | | **All** | **SVR** | **Others^{a}** | **p** |
|---|---|---|---|---|---|
| Age (years) | | 42.8 (36.1-52.9) | 40.4 (35.5-48.9) | 44.4 (36.8-57.3) | 0.002 |
| Male sex (%) | | **235** (63.0) | **95** (68.3) | **140** (59.8) | 0.120 |
| Genotype 1 (%) | | **247** (66.2) | **69** (49.6) | **178** (76.1) | <0.001 |
| Metavir F (%): | | | | | 0.220 |
| | - 0 | **13** (3.5) | **4** (2.9) | 9 (3.8) | |
| | - 1 | **147** (39.4) | **58** (41.7) | **89** (38.0) | |
| | - 2 | **108** (29.0) | **43** (30.9) | **65** (27.8) | |
| | - 3 | **56** (15.0) | **22** (15.8) | **34** (14.5) | |
| | - 4 | **49** (13.1) | **11** (7.9) | **38** (16.2) | |
| ALT (IU/L) | | 73 (48-120) | 82 (53-121) | 68 (45-115) | 0.093 |
| Prothrombin time (%) | | 95 (89-102) | 95 (89-101) | 96 (89-103) | 0.593 |
| Bilirubin (µmol/L) | | 10 (7-13) | 9 (7-13) | 10 (7-13) | 0.376 |
| Albumin (g/L) | | 44.0 (41.7-47.1) | 44.7 (42.3-47.1) | 44.0 (41.5-47.2) | 0.168 |
| ***Follow-up:*** | | | | | |
| | - Significant liver-related event (%) ^{b} | **47** (12.6) | **5** (3.6) | **42** (17.9) | <0.001 |
| | - Death (all causes, %) | **56** (15.0) | **6** (4.3) | **50** (21.4) | <0.001 |
| | - Liver-related death (%) | **23** (6.2) | **0** (0.0) | **23** (9.8) | <0.001 |

| | | | | | |
|---|---|---|---|---|---|
| Quantitative variables are expressed as median with 1 and 3^{rd} quartiles in brackets, and categorical variables as absolute number with percentage into brackets. SVR: sustained viral response. ^{a} Either no or unsuccessful antiviral therapy ^{b} Only the first significant liver-related event during follow-up was taken into account | | | | | |

Median liver biopsy length at inclusion was 20mm (1^{st} quartile: 15mm, 3^{rd} quartile: 25mm). 47 patients (12.6%) experienced at least one SLRE during follow-up. First SLRE were: large varices (n=20), hepatocellular carcinoma (n=13), ascites (n=4), jaundice (n=4), hepatic encephalopathy (n=1), variceal bleeding (n=1), ascites and jaundice (n=2), ascites and jaundice and encephalopathy (n=2). Median follow-up for SLRE was 7.6 years (interquartile range: 4.0-12.1; 2972 person-years). Cumulative incidence of first SLRE at median follow-up was 11.5% **(****Figure 6a****).**

50 patients died during follow-up; cause of death was unknown in 2 cases and liver-related in 17. Also, 6 patients had liver transplantation. Death was thus considered as liver-related in 23 patients. As death occurred after the first SLRE, the median follow-up for death was longer than for SLRE: 9.5 years (interquartile range: 5.8-13.0; 3508 person-years). Survival without liver-related death and overall survival at median follow-up were, respectively: 95.1% **(****Figure 6b****)** and 88.8%.

37.2% of patients achieved SVR during follow-up, and 62.8% had either no or unsuccessful antiviral therapy. There was no liver-related death during the follow-up of patients who achieved SVR, and only 5 of them had a SLRE. 3 of these 5 patients (2 with Metavir F4 and 1 with F3 at inclusion) developed large esophageal varices before achieving SVR. One patient (Metavir F2 at inclusion) had large esophageal varices 5 years after achieving SVR in a context of metabolic syndrome, and thus attributed to an ongoing active nonalcoholic steatohepatitis. The last patient (Metavir F3 at inclusion) had jaundice and ascites before achieving SVR in a context of recovery of excessive alcohol consumption during follow-up.

### Prediction of first SLRE

### Liver biopsy versus blood tests

**Figure 7a** shows the cumulative incidence of first SLRE as a function of Metavir F stages. The difference was highly significant between F4 and F3 stages (p<0.001), but not among F0 to F3 stages (p=0.174).

Harrell C-indexes of liver biopsy (Metavir F) and blood fibrosis tests for the prediction of the first SLRE are depicted in **Table 9** (paired comparisons between tests are detailed in **Table 10).**

**Table 9: Harrell C-indexes of liver biopsy (Metavir F) and blood fibrosis tests for the prediction of the first significant liver-related event or liver-related death**

| **Test** | **First significant liver-related event** | **Liver-related death** |
|---|---|---|
| Metavir F | 0.811 [0.751-0.868] **^{a}** | 0.849 [0.755-0.920] ^{b} |
| APRI | 0.861 [0.813-0.902] | 0.869 [0.793-0.932] |
| FIB4 | 0.879 [0.832-0.919] | 0.911 [0.851-0.954] |
| Fibrotest | 0.838 [0.776-0.897] | 0.856 [0.765-0.932] |
| Hepascore | 0.809 [0.729-0.880] | 0.894 [0.811-0.951] |
| FibroMeter ^{V2G} | 0.870 [0.812-0.922] | 0.898 [0.842-0.945] |
| CirrhoMeter^{V2G} | 0.835 [0.762-0.902] | 0.884 [0.795-0.952] |

| | | |
|---|---|---|
| **^{a}** Comparison of Metavir F vs blood tests: p=0.039 vs APRI, p=0.002 vs FIB4, p=0.005 vs FibroMeter^{v2G}, p>0.340 for other comparisons ^{b} Comparison of Metavir F vs blood tests: p=0.011 vs FIB4, p=0.078 vs FibroMeter^{v2G}, p>0.190 for other comparisons | | |

**Table 10: Paired comparisons of Harrell C-indexes of blood fibrosis tests and liver biopsy (Metavir F) for the prediction of first significant liver-related event or liver-related death**

| | **First significant liver-related event** | **Liver-related death** |
|---|---|---|
| Metavir F | 0.811 [0.751-0.868] | 0.849 [0.755-0.920] |
| APRI | 0.861 [0.813-0.902] | 0.869 [0.793-0.932] |
| FIB4 | 0.879 [0.832-0.919] | 0.911 [0.851-0.954] |
| Fibrotest | 0.838 [0.776-0.897] | 0.856 [0.765-0.932] |
| Hepascore | 0.809 [0.729-0.880] | 0.894 [0.811-0.951] |
| FibroMeter V | 0.870 [0.812-0.922] | 0.898 [0.842-0.945] |
| CirrhoMeter V | 0.835 [0.762-0.902] | 0.884 [0.795-0.952] |

| Comparison (p) : | | |
|---|---|---|
| Metavir F vs APRI | **0.039** | 0.510 |
| Metavir F vs FIB4 | **0.002** | **0.011** |
| Metavir F vs Fibrotest | 0.420 | 0.730 |
| Metavir F vs Hepascore | 0.880 | 0.200 |
| Metavir F vs FibroMeter V^{V2G} | **0.005** | 0.078 |
| Metavir F vs CirrhoMeter V^{V2G} | 0.340 | 0.190 |
| APRI vs FIB4 | 0.240 | 0.064 |
| APRI vs Fibrotest | 0.350 | 0.680 |
| APRI vs Hepascore | 0.130 | 0.530 |
| APRI vs FibroMeter V^{V2G} | 0.460 | 0.210 |
| APRI vs CirrhoMeter V^{V2G} | 0.400 | 0.640 |
| FIB4 vs Fibrotest | 0.100 | **0.049** |
| FIB4 vs Hepascore | **0.040** | 0.590 |
| FIB4 vs FibroMeter V^{V2G} | 0.680 | 0.480 |
| FIB4 vs CirrhoMeter V^{V2G} | 0.140 | 0.230 |
| Fibrotest vs Hepascore | 0.200 | 0.150 |
| Fibrotest vs FibroMeter V^{V2G} | **0.032** | 0.089 |
| Fibrotest vs CirrhoMeter V^{V2G} | 0.940 | **0.053** |
| Hepascore vs FibroMeter V^{V2G} | **0.028** | 0.870 |
| Hepascore vs CirrhoMeter V^{V2G} | 0.140 | 0.640 |
| FibroMeter V^{V2G} vs CirrhoMeter V^{V2G} | 0.076 | 0.430 |

| | | |
|---|---|---|
| Significant differences in bold | | |

Briefly, Metavir F and the 6 blood fibrosis tests had good discriminative ability for the prediction of the first SLRE (with C-indexes >0.8). 5 out 6 blood fibrosis tests had a higher C-index than liver biopsy; this increase being significant for FIB4 (p=0.002), FibroMeter (p=0.005), and APRI (p=0.039).

To evaluate each blood fibrosis test against liver biopsy, we performed successive multivariate stepwise forward Cox models including each blood test with Metavir F, adjusted on age, sex, HCV genotype (1 vs others) and SVR for the prediction of first SLRE **(Table 11).**

**Table 11: Evaluation of blood fibrosis tests against Metavir F staging for the prediction of liver-related outcomes. Each score was separately introduced with Metavir F stage in a multivariate stepwise forward Cox model adjusted on age, sex, sustained viral response, and HCV genotype (1 vs others).**

| **Test** | **First significant liver-related event** | | | **Liver-related death** | | |
|---|---|---|---|---|---|---|
| | **Step** | **Variable** | **p** | **Step** | **Variable** | **p** |
| APRI | 1^{st} | APRI | <0.001 | 1^{st} | APRI | <0.001 |
| | 2^{nd} | Metavir F | <0.001 | 2^{nd} | Metavir F | 0.003 |
| FIB4 | 1^{st} | FIB4 | <0.001 | 1^{st} | FIB4 | <0.001 |
| | 2^{nd} | Metavir F | <0.001 | 2^{nd} | Metavir | 0.002 |
| Fibrotest | 1^{st} | Fibrotest | <0.001 | 1^{st} | Metavir F | <0.001 |
| | 2^{nd} | Metavir F | 0.027 | 2^{nd} | - | - |
| Hepascore | 1^{st} | Metavir F | <0.001 | 1^{st} | Metavir F | <0.001 |
| | 2^{nd} | Hepascore | 0.022 | 2^{nd} | Hepascore | 0.028 |
| FibroMeter^{V2G} | 1^{st} | FibroMeter^{V2G} | <0.001 | 1^{st} | Metavir F | <0.001 |
| | 2^{nd} | - | - | 2^{nd} | - | - |
| CirrhoMeter^{V2G} | 1^{st} | CirrhoMeter^{V2G} | <0.001 | 1^{st} | CirrhoMeter^{2GV} | <0.001 |
| | 2^{nd} | - | - | 2^{nd} | - | - |

Each blood fibrosis test was an independent predictor of SLRE (at the first step, except for Hepascore). Metavir F was not selected as an independent predictor of SLRE when introduced with FibroMeter or CirrhoMeter in the multivariate analysis. This showed that FibroMeter or CirrhoMeter entirely caught the independent prediction without any significant additional prediction by liver biopsy.

Taken together, all the previous results show that blood fibrosis tests are predictors at least as accurate, and for some significantly better than Metavir F staging for the prediction of SRLE in CHC patients.

### Fibrosis classifications

As our results showed blood fibrosis tests as prognostic markers, they should individualize subgroups of patients with different prognosis. A robust *a posteriori* determination of these subgroups would require a large cohort (≥1000 patients) with high rate of events, followed by external validation. We thus chose to evaluate the prognostic significance of blood tests fibrosis classifications. This was relevant for two reasons. First, fibrosis classifications allow for an *a priori* stratification of patients. Second, fibrosis classifications have been developed to estimate fibrosis stages and this original design would validate their ability to identify at-risk patients.

**Figures 7b to 7d** show the cumulative incidence of first SLRE as a function of Fibrotest, FibroMeter, or CirrhoMeter fibrosis classifications. Unlike for Fibrotest, the increase in FibroMeter or CirrhoMeter classes was associated with a progressively worsening prognosis. Moreover, patients included in the FM₁ or CM₁ classes (16.7% of patients in each) had excellent prognoses with no SLRE during follow-up. FIB4 provided the highest Harrell C-index for the prediction of first SLRE, but detailed fibrosis classification is not available for this blood test. We thus evaluated the prognosis of 3 subgroups defined by its 2 recommended 1.45 and 3.25 diagnostic cut-offs. Interestingly, these 3 subgroups had significant different prognosis (p<0.001, **Figure 8****).** However, 11 patients (4.6%) included in the subgroup with putative best prognosis (i.e., FIB4 < 1.45) experienced SLRE during follow-up.

### How to predict SLRE in clinical practice?

We have already shown in cross-sectional studies that combination of fibrosis tests by binary logistic regression improves the non-invasive diagnosis of liver fibrosis (Boursier et al, Am J Gastroenterol 2011; 106:1255-63). We used the same design in the present longitudinal study to determine the most complementary combination of blood fibrosis tests for the prediction of first-SLRE.

In multivariate forward Cox model including the 6 blood fibrosis tests, age, sex, SVR, and HCV genotype (1 vs others), independent predictors of first SLRE were CirrhoMeter (p<0.001), FibroMeter (p=0.008), and SVR (p=0.010). This result was not surprising as FibroMeter and CirrhoMeter, the first dedicated to significant fibrosis and the latter to cirrhosis, are clearly complementary with CirrhoMeter "stretching" high FibroMeter values **(****Figure 9****).** The interaction between FibroMeter and CirrhoMeter was significant in the multivariate analysis (p=0.002). In addition, the cut-off between FM₄ and FM₅ classes clearly distinguished two groups: FM₁₋₄ classes displayed a larger range of FibroMeter values than CirrhoMeter values, and the opposite was observed in FM₅₋₇ classes **(****Figure 9****).** We thus repeated the multivariate analysis separately in FM₁₋₄ and FM₅₋₇ groups. Multivariate backward Cox model including SVR, FibroMeter, and CirrhoMeter showed FibroMeter (p=0.024) and SVR (p=0.040) as independent predictors of first SLRE in FM₁₋₄ group. By contrast, CirrhoMeter (p<0.001) was the only independent predictor in FM₅₋₇ group. These results suggested that FibroMeter better predicted first SLRE in the early stages of CHC, whereas CirrhoMeter was a better predictor in patients with advanced disease.

These results lead us to evaluate a combination of FibroMeter classification for patients included in the FM₁₋₄ classes with CirrhoMeter classification for patients included in FM₅₋₇ classes. This combination provided 10 intermediate classes (FM₁₋₄ and CM₁₋₆ if FM₅₋₇). These 10 intermediate classes were grouped into 5 final classes owing to significantly different prognosis (FMi, FM_{2/3}, FM_{≥4} + CM_{≤4}, FM_{≥4} + CM₅, FM_{≥4} + CM₆) defining the new FM/CM classification. The rates of patients included in the 5 classes of the FM/CM classification were, respectively from the 1^{st} to the 5^{th}: 16.7%, 41.5%, 27.2%, 10.0%, and 4.6%.

### FM/CM classification versus liver biopsy

As with Metavir F staging, FM/CM classification split patients into 5 classes but provided several advantages. First, the cumulative incidence of first SLRE during follow-up was significantly different between adjacent classes of the FM/CM classification **(****Figure 10a****)** whereas there was no significant difference between Metavir F0 to F3 stages **(****Figure 7a****).** Second, no SLRE occurred during the follow-up of patients included in the first class of the FM/CM classification whereas 1 F0 patient according to baseline liver biopsy had jaundice during follow-up. Third, patients included in the highest class of the FM/CM classification had a markedly worse prognosis **(****Figure 10a****)** than cirrhotic patients as defined by liver biopsy **(****Figure 7a****).** Finally, in multivariate stepwise forward Cox Model including Metavir F, FibroMeter, CirrhoMeter, and FM/CM classifications, FM/CM classification was selected at the first step of the multivariate analysis as independent predictor of SLRE.

### Liver-related death

Survival without liver-related death was significantly different between Metavir F4 and F3 stages (p<0.001), but not among F0 to F3 stages (p=0.456, **Figure 11a****).** All blood fibrosis tests had a higher C-index than liver biopsy **(Table 9);** this increase was significant for FIB4 (p=0.011, **Table 10).** Multivariate stepwise forward Cox models including successively each blood test with Metavir F staging **(Table 11)** showed that CirrhoMeter was the only blood fibrosis test with no independent role for Metavir F. Increasing classes of FibroMeter or CirrhoMeter classification was associated with a progressive increase in liver-related death during follow-up **(****Figure 11****),** and the highest class of CirrhoMeter classification identified a subgroup of very high risk patients. As for first SLRE, FIB4 provided the highest Harrell C-index for the prediction of liver-related death and, using the recommended diagnostic cut-offs, FIB4 identified 3 subgroups with significant different prognoses **(****Figure 8b****).** 3 patients (1.3%) included in the subgroup with putative best prognosis (i.e., FIB4 < 1.45) died from liver-related cause during follow-up.

### Evaluation of liver-related death in clinical practice

In a multivariate forward Cox model including the 6 blood fibrosis tests, age, sex, and HCV genotype (SVR was not introduced in the model as there was no death in SVR patients), CirrhoMeter was the only independent predictor (p<0.001) of liver-related-death. No liver-related death occurred among patients included in CM₁ class **(****Figure 11d****)**. Nevertheless, survival without liver-related death was not significantly different between the adjacent classes from CM₁ to CM₅; it was significantly different only between CM₅ and CM₆ classes.

By contrast, survival without liver-related death was significantly different between the 2^{nd} and the 3^{rd} classes, and between the 4^{th} and the 5^{th} classes of the FM/CM classification **(****Figure 10b****).** Finally, in a multivariate stepwise forward Cox Model including Metavir F, FibroMeter, CirrhoMeter, and FM/CM classifications, FM/CM classification was the only independent predictor of liver-related death.

### Evaluation of liver prognosis in clinical practice

To summarize, liver-prognosis in CHC is best assessed by a combination of two complementary tests: the FibroMeter (targeted for fibrosis) in patients with mild disease and CirrhoMeter (targeted for cirrhosis) in patients with advanced disease. Both tests provide a combined FM/CM classification which is the best independent predictor of first SLRE as well as liver-related death. The incidences of 5 and 10-year SLRE or liver-related death as a function of FM/CM classification are presented in **Figure 12** (details in **Table 12).**

**Table 12: Cumulative incidence (%) of first significant liver-related event (SLRE) or liver-related death as a function of the 5 classes of the FM/CM classification.**

| | | **Classes of FM/CM classification** | | | | |
|---|---|---|---|---|---|---|
| | | **1 (FM₁)** | **2 (FM_{2/3})** | **3 (FM_{≥4} &CM_{≤4})** | **4 (FM_{≥4} &CM₅)** | **5 (FM_{≥4} &CM₆)** |
| 5-year SLRE | All | 0.0 | 0.7 | 4.5 | 18.2 | 76.5 |
| | No SVR^{a} | 0.0 | 1.3 | 4.0 | 23.1 | 76.5 |
| | SVR | 0.0 | 0.0 | 5.6 | 8.3 | -^{b} |
| 10-year SLRE | All | 0.0 | 6.0 | 15.7 | 29.8 | 94.1 |
| | No SVR^{a} | 0.0 | 10.9 | 23.1 | 35.1 | 94.1 |
| | SVR | 0.0 | 1.8 | 5.6 | 19.8 | -^{b} |
| 5-year liver death | All | 0.0 | 0.0 | 0.0 | 0.0 | 39.1 |
| | No SVR^{a} | 0.0 | 0.0 | 0.0 | 0.0 | 39.1 |
| | SVR | 0.0 | 0.0 | 0.0 | 0.0 | -^{b} |
| 10-year | All | 0.0 | 0.0 | 7.0 | 7.5 | 59.4 |
| liver death | No SVR^{a} | 0.0 | 0.0 | 12.3 | 10.9 | 59.4 |
| | SVR | 0.0 | 0.0 | 0.0 | 0.0 | -^{b} |

| | | | | | | |
|---|---|---|---|---|---|---|
| ^{a} Either no or unsuccessful antiviral therapy ^{b} no patient in this case | | | | | | |

Our results demonstrate that blood fibrosis tests are globally at least as accurate as Metavir F staging for the prediction of liver prognosis in CHC patients. Indeed, none of the blood fibrosis tests had a significantly lower Harrell C-index than liver biopsy for the prediction of SLRE or liver-related death. In multivariate analyses including each blood test with Metavir F staging, almost all blood fibrosis tests were independent predictors of SLRE or liver-related death, suggesting that blood fibrosis tests provide relevant prognostic information. Moreover, FibroMeter and CirrhoMeter were independent predictors of SLRE with no role for Metavir F, as was CirrhoMeter for liver-related death.

Among the 6 blood fibrosis tests evaluated, multivariate analysis identified FibroMeter and CirrhoMeter as the best combination for the prediction of first SLRE. These two tests, the former specifically targeted for significant fibrosis and the latter for cirrhosis (Boursier et al, Eur J Gastroenterol Hepatol 2009; 21:28-38), are very complementary **(****Figure 9****)** and their association improves the prediction of first SLRE: FibroMeter is more predictive at low fibrosis levels and CirrhoMeter at high fibrosis levels since it amplifies the test scale like a two-speed gearbox. Finally, similar to the Child-Pugh score or hepatic venous pressure gradient measurement in cirrhotic patients, CirrhoMeter can stratify high FibroMeter values to improve the identification of subgroups with significantly different prognoses. In clinical practice, FibroMeter and CirrhoMeter include the same markers, but with different coefficients, and can be thus simultaneously calculated with no additional costs. Because it requires a very large population to robustly determine *a posteriori* prognostic subgroups, we decided to stratify patients according to the *a priori* fibrosis classifications or diagnostic cut-offs that are currently used in clinical practice. In this setting, the combination of FibroMeter and CirrhoMeter fibrosis classifications in the FM/CM classification has several advantages compared to Metavir F staging. First, whereas Metavir F staging identified only two groups of patients with different prognoses (F0-3 vs F4), FM/CM classification identified 5 significantly different groups **(****Figure 10a****).** Second, contrary to Metavir F staging, no SLRE occurred in the lower class of FM/CM classification, defining thus a subgroup with excellent prognosis. Third, the highest class of FM/CM identified a subgroup of very high-risk patients that was not identified by Metavir F staging.

FIB4, an inexpensive and easy-to-calculate blood fibrosis test, showed very good prognostic accuracy in our study. It was not selected as an independent predictor of first SLRE by multivariate analysis, probably because it was masked by the high complementary role of FibroMeter and CirrhoMeter for this endpoint. By using its 2 recommended diagnostic cut-offs, FIB4 identified 3 subgroups of patients whose prognosis was significantly different. However, 11 patients from the best-prognosis subgroup experienced SLRE during their follow-up whereas, by comparison, no patients included in the first class of FibroMeter or CirrhoMeter classification had SLRE. This could be due to an inadequate choice for FIB4 thresholds. Indeed, no SLRE occurred during follow-up of patients included in the 3 first deciles of FIB4. This suggests that FIB4 is also able to determine a subgroup of patients with excellent prognosis, and FIB4 thresholds should be refined for this purpose.

In conclusion, blood fibrosis tests are also prognostic tests at least as accurate as liver biopsy for the prediction of liver prognosis in patients with CHC. A combination of two complementary blood tests, one targeted for fibrosis (FibroMeter) and the other for cirrhosis (CirrhoMeter), is more accurate than liver biopsy and appears as a simple and accurate method for the baseline assessment of prognosis in clinical practice.

## Claims

1. An *in vitro* non-invasive prognostic method for assessing the risk of death and/or of liver-related event in a subject, comprising:
a. obtaining at least three variables selected from age, sex, weight, alpha-2 macroglobulin (A2M), hyaluronic acid (HA), prothrombin index (PI), platelets (PLT), aspartate aminotransferase (AST), urea, gamma-glutamyl transpeptidase (GGT), alanine aminotransferase (ALT), ferritin (Fer), and glucose (Glu);
b. obtaining at least one blood test result by multivariate combination, with a binary logistic regression, of the at least three variables obtained in step (a), said blood test being a FibroMeter;
c. obtaining at least one elastometry data from medical imaging or clinical measurement; and
d. mathematically combining in a multivariate time-related model:
- said at least three variables obtained in step (a) and/or said at least one blood test result obtained in step (b); and
- said at least one elastometry data obtained in step (c);
thereby obtaining a prognostic score.

2. The *in vitro* non-invasive prognostic method according to claim **1,** wherein said at least one elastometry data is liver stiffness measurement (LSM).

3. The *in vitro* non-invasive prognostic method according to claim **2,** wherein said LSM is measured by Vibration Controlled Transient Elastography (VCTE).

4. The *in vitro* non-invasive prognostic method according to any one of claims **1** to **3,** comprising mathematically combining in a multivariate time-related model in step (d) the following variables, or at least the following variables: a blood test result, said blood test being a FibroMeter, LSM, age, and sex.

5. The *in vitro* non-invasive prognostic method according to any one of claims **1** to **4,** wherein said multivariate time-related model is a Cox model, said multivariate Cox model being time-fixed or time-dependent.

6. The *in vitro* method according to claim **5,** wherein said multivariate Cox model is time-dependent.

7. The *in vitro* method according to any one of claims **1** to **6,** wherein death is all-cause death and/or liver-related death.

8. The *in vitro* method according to any one of claims **1** to **7,** wherein said liver-related event is selected from ascites, encephalopathy, jaundice, occurrence of large esophageal varices, variceal bleeding, gastro-intestinal hemorrhage, hepato-renal syndrome, hepatocellular carcinoma, hepatic transplantation, esophageal varices, and portal hypertension.

9. The *in vitro* method according to any one of claims **1** to **8,** wherein the subject is affected with a liver disease and/or the subject is a patient affected with a chronic disease.

10. The *in vitro* method according to claim **9,** wherein the subject is affected with a liver disease selected from significant porto-septal fibrosis, severe porto-septal fibrosis, centrolobular fibrosis, cirrhosis, perisinusoidal fibrosis, the fibrosis being of alcoholic or non-alcoholic origin.

11. The *in vitro* method according to claim **9,** wherein the subject is a patient affected with a chronic disease selected from chronic viral hepatitis C, chronic viral hepatitis B, chronic viral hepatitis D, chronic viral hepatitis E, non-alcoholic fatty liver disease (NAFLD), alcoholic chronic liver disease, autoimmune hepatitis, primary biliary cirrhosis, hemochromatosis and Wilson disease.

12. The *in vitro* method according to any one of claims **1** to **11,** being computer implemented.

13. A microprocessor comprising a computer algorithm for carrying out the *in vitro* method according to any one of claims **1** to **12.**

## Patentansprüche

1. Nicht-invasives prognostisches *In-vitro*-Verfahren zum Bewerten des Risikos von Tod und/oder eines leberbezogenen Ereignisses bei einem Subjekt, umfassend:
a. Erhalten von mindestens drei Variablen, ausgewählt aus Alter, Geschlecht, Gewicht, Alpha-2-Makroglobulin (A2M), Hyaluronsäure (HA), Prothrombin-Index (PI), Thrombozyten (PLT), Aspartat-Aminotransferase (AST), Harnstoff, Gamma-Glutamyl-Transpeptidase (GGT), Alanin-Aminotransferase (ALT), Ferritin (Fer) und Glucose (Glu);
b. Erhalten mindestens eines Bluttestergebnisses durch multivariate Kombination, mit einer binären logistischen Regression, der mindestens drei in Schritt (a) erhaltenen Variablen, wobei der Bluttest ein FibroMeter ist;
c. Erhalten mindestens eines Elastometriedatenwerts aus medizinischer Bildgebung oder klinischer Messung; und
d. mathematisches Kombinieren, in einem multivariaten zeitbezogenen Modell:
- der mindestens drei in Schritt (a) erhaltenen Variablen und/oder des mindestens einen in Schritt (b) erhaltenen Bluttestergebnisses; und
- des mindestens einen in Schritt (c) erhaltenen Elastometriedatenwerts;
wodurch ein prognostischer Score erhalten wird.

2. Nicht-invasives prognostisches *In-vitro*-Verfahren nach Anspruch **1,** wobei der mindestens eine Elastometriedatenwert eine Lebersteifigkeitsmessung (LSM) ist.

3. Nicht-invasives prognostisches *In-vitro*-Verfahren nach Anspruch **2,** wobei die LSM durch vibrationsgesteuerte transiente Elastographie (VCTE) gemessen wird.

4. Nicht-invasives prognostisches *In-vitro*-Verfahren nach einem der Ansprüche **1** bis **3,** das mathematische Kombinieren, in einem multivariaten zeitbezogenen Modell in Schritt (d), der folgenden Variablen oder mindestens der folgenden Variablen umfasst: ein Bluttestergebnis, wobei der Bluttest ein FibroMeter ist, LSM, Alter und Geschlecht.

5. Nicht-invasives prognostisches *In-vitro*-Verfahren nach einem der Ansprüche **1** bis **4,** wobei das multivariate zeitbezogene Modell ein Cox-Modell ist, wobei das multivariate Cox-Modell zeitlich festgelegt oder zeitabhängig ist.

6. *In-vitro*-Verfahren nach Anspruch **5,** wobei das multivariate Cox-Modell zeitabhängig ist.

7. *In-vitro*-Verfahren nach einem der Ansprüche **1** bis **6,** wobei es sich bei Tod um Tod jeglicher Ursache und/oder leberbedingten Tod handelt.

8. *In-vitro-*Verfahren nach einem der Ansprüche **1** bis **7,** wobei das leberbezogene Ereignis ausgewählt ist aus Aszites, Enzephalopathie, Gelbsucht, Auftreten von großen Ösophagusvarizen, Varizenblutung, gastrointestinaler Blutung, hepatorenalem Syndrom, hepatozellulärem Karzinom, Lebertransplantation, Ösophagusvarizen und portaler Hypertonie.

9. *In-vitro*-Verfahren nach einem der Ansprüche **1** bis **8,** wobei das Subjekt an einer Lebererkrankung leidet und/oder das Subjekt ein Patient ist, der an einer chronischen Erkrankung leidet.

10. *In-vitro*-Verfahren nach Anspruch **9,** wobei das Subjekt an einer Lebererkrankung leidet, ausgewählt aus signifikanter portoseptaler Fibrose, schwerer portoseptaler Fibrose, zentrilobulärer Fibrose, Zirrhose, perisinusoidaler Fibrose, wobei die Fibrose alkoholischen oder nichtalkoholischen Ursprungs ist.

11. *In-vitro*-Verfahren nach Anspruch **9,** wobei das Subjekt ein Patient ist, der an einer chronischen Erkrankung leidet, ausgewählt aus chronischer Virushepatitis C, chronischer Virushepatitis B, chronischer Virushepatitis D, chronischer Virushepatitis E, nichtalkoholischer Fettlebererkrankung (NAFLD), alkoholischer chronischer Lebererkrankung, Autoimmunhepatitis, primärer biliärer Zirrhose, Hämochromatose und Morbus Wilson.

12. *In-vitro*-Verfahren nach einem der Ansprüche **1** bis **11,** das computerimplementiert ist.

13. Mikroprozessor, der einen Computeralgorithmus zum Ausführen des *In-vitro-*Verfahrens nach einem der Ansprüche **1** bis **12** umfasst.

## Revendications

1. Méthode pronostique *in vitro* non-invasive pour évaluer le risque de décès et/ou d'événement lié au foie chez un sujet, comprenant
a. obtenir au moins trois variables sélectionnées parmi âge, sexe, poids, alpha-2 macroglobuline (A2M), acide hyaluronique (HA), taux de prothrombine (PI), plaquettes (PLT), aspartate aminotransférase (AST), urée, gamma-glutamyl transpeptidase (GGT), alanine aminotransférase (ALT), ferritine (Fer) et glucose (Glu) ;
b. obtenir au moins un résultat de test sanguin par combinaison multivariée, avec une régression logistique binaire, des au moins trois variables obtenues à l'étape a), ledit test sanguin étant un FibroMeter ;
c. obtenir au moins une donnée d'élastométrie à partir d'une imagerie médicale ou d'une mesure clinique ; et
d. combiner mathématiquement dans un modèle multivarié lié au temps :
- lesdites au moins trois variables obtenues à l'étape (a) et/ou ledit au moins un résultat de test sanguin obtenu à l'étape (b) ; et
- ladite au moins une donnée d'élastométrie obtenue à l'étape (c) ;
obtenant ainsi un score pronostique.

2. La méthode pronostique *in vitro* non-invasive selon la revendication **1,** dans laquelle ladite au moins une donnée d'élastométrie est une mesure de la rigidité du foie (*LSM - liver stiffness measurement).*

3. La méthode pronostique *in vitro* non-invasive selon la revendication **2,** dans laquelle ladite *LSM* est mesurée par élastographie impulsionnelle à vibration contrôlée (VCTE).

4. La méthode pronostique *in vitro* non-invasive selon l'une quelconque des revendications **1** à **3,** comprenant la combinaison mathématique dans un modèle multivarié lié au temps à l'étape (d) des variables suivantes, ou au moins des variables suivantes : un résultat de test sanguin, ledit test sanguin étant un FibroMètre, *LSM,* âge et sexe.

5. La méthode pronostique *in vitro* non-invasive selon l'une quelconque des revendications **1** à **4,** dans laquelle ledit modèle multivarié lié au temps est un modèle de Cox, ledit modèle de Cox multivarié étant fixé dans le temps ou dépendant du temps.

6. La méthode *in vitro* selon la revendication 5, dans laquelle ledit modèle de Cox multivarié est dépendant du temps.

7. La méthode *in vitro* selon l'une quelconque des revendications **1** à **6,** dans laquelle le décès est un décès toutes causes confondues et/ou un décès lié au foie.

8. La méthode *in vitro* selon l'une quelconque des revendications **1** à **7,** dans laquelle ledit événement lié au foie est sélectionné parmi les ascites, l'encéphalopathie, la jaunisse, l'apparition de grosses varices oesophagiennes, le saignement variqueux, l'hémorragie gastro-intestinale, le syndrome hépatorénal, le carcinome hépatocellulaire, la transplantation hépatique, les varices oesophagiennes et l'hypertension portale.

9. La méthode *in vitro* selon l'une quelconque des revendications **1** à **8,** dans laquelle le sujet est atteint d'une maladie du foie et/ou le sujet est un patient atteint d'une maladie chronique.

10. La méthode *in vitro* selon la revendication **9,** dans laquelle le sujet est atteint d'une maladie du foie sélectionnée parmi la fibrose porto-septale significative, la fibrose porto-septale sévère, la fibrose centrolobulaire, la cirrhose, la fibrose périsinusoïdale, la fibrose étant d'origine alcoolique ou non alcoolique.

11. La méthode *in vitro* selon la revendication **9,** dans laquelle le sujet est un patient atteint d'une maladie chronique sélectionnée parmi l'hépatite virale C chronique, l'hépatite virale B chronique, l'hépatite virale D chronique, l'hépatite virale E chronique, la maladie du foie gras non alcoolique (NAFLD), la maladie alcoolique chronique du foie, l'hépatite auto-immune, la cirrhose biliaire primitive, l'hémochromatose et la maladie de Wilson.

12. La méthode *in vitro* selon l'une quelconque des revendications **1** à **11,** étant mise en oeuvre par ordinateur.

13. Microprocesseur comprenant un algorithme informatique pour mettre en oeuvre la méthode *in vitro* selon l'une quelconque des revendications **1** à **12.**
